(19) Europäisches Patentamt
European Patent Office
Office européen des brevets

(11) EP 4 653 450 A1

(12) **EUROPEAN PATENT APPLICATION**
published in accordance with Art. 153(4) EPC

(43) Date of publication:
**26.11.2025 Bulletin 2025/48**

(21) Application number: **24744194.2**

(22) Date of filing: **15.01.2024**

(51) International Patent Classification (IPC):
***C07K 5/027*** *(2006.01)* ***C07D 285/01*** *(2006.01)*
***C07D 417/14*** *(2006.01)* ***C07C 51/43*** *(2006.01)*
***A61K 31/381*** *(2006.01)* ***A61K 31/41*** *(2006.01)*
***A61P 31/14*** *(2006.01)*

(52) Cooperative Patent Classification (CPC):
**A61K 31/381; A61K 31/41; A61P 31/14; C07C 51/43; C07D 285/01; C07D 417/14; C07K 5/0205**

(86) International application number:
**PCT/CN2024/072253**

(87) International publication number:
**WO 2024/153018 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.01.2023 CN 202310064780**

(71) Applicant: **Hainan Simcere Pharmaceutical Co., Ltd.**
**Haikou, Hainan 570311 (CN)**

(72) Inventors:
- **ZHANG, Lei**
  **Nanjing, Jiangsu 210042 (CN)**
- **ZHOU, Feng**
  **Shanghai 201318 (CN)**
- **SONG, Wei**
  **Nanjing, Jiangsu 210042 (CN)**
- **LI, Yinqiang**
  **Nanjing, Jiangsu 210042 (CN)**
- **CUI, Nan**
  **Nanjing, Jiangsu 210042 (CN)**
- **GAO, Xiaofang**
  **Nanjing, Jiangsu 210042 (CN)**
- **JIANG, Lei**
  **Shanghai 201318 (CN)**
- **TANG, Renhong**
  **Shanghai 201318 (CN)**

(74) Representative: **Mewburn Ellis LLP**
**Aurora Building**
**Counterslip**
**Bristol BS1 6BX (GB)**

# (54) CRYSTAL OF SPIRO COMPOUND AND PREPARATION METHOD THEREFOR

(57) The present disclosure relates to a crystal form of a spiro compound represented by formula (I), a preparation method therefor, and use thereof in treating diseases caused by coronavirus infection and/or small RNA virus infection.

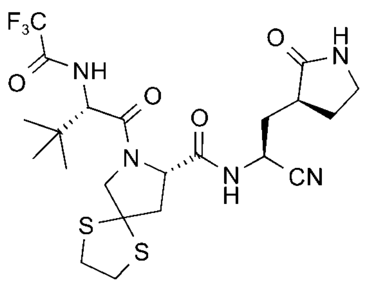

formula (I).

EP 4 653 450 A1

## Description

## CROSS-REFERENCE TO RELATED APPLICATION

The present application claims the priority to and benefit of the Chinese Patent Application No. 202310064780.5 filed with China National Intellectual Property Administration on January 16, 2023, which is incorporated herein by reference in its entirety.

## TECHNICAL FIELD

The present disclosure relates to the field of pharmaceutical chemistry, and particularly to a crystal form of a spiro compound, a preparation method therefor, and a pharmaceutical composition and use containing the same.

## BACKGROUND

Coronaviruses are single-stranded positive-sense RNA viruses. Some coronaviruses can spread widely in the human population and can cause severe symptoms. There are 7 coronaviruses known to be capable of infecting humans, namely HCoV-229E, HCoV-OC43, HCoV-NL63, HCoV-HKU1, SARS-CoV, MERS-CoV, and SARS-CoV-2. Most functional proteins of coronaviruses are encoded by ORF1ab genes, and they are first translated into a polyprotein and then cleaved by 3CL protease and PL protease into multiple active proteins. Therefore, inhibiting the activity of 3CL protease can effectively inhibit the replication of the viruses. Different coronavirus 3CL proteases have high structural homology. Therefore, in general, 3CL protease inhibitors have broad-spectrum anti-coronavirus activity.

In addition to coronaviruses, 3CL protease also plays an important role in the proteolysis of polyproteins encoded by picornaviruses. 3CL protease inhibitors can effectively inhibit the replication of picornaviruses. Enterovirus 71 is a picornavirus, which is one of the common viruses causing hand, foot and mouth disease, and may also cause various diseases such as meningitis, brainstem encephalitis, myocarditis, and the like. In recent years, enterovirus 71 has exploded many times in the population of infants and young children, and there is still a lack of efficient therapeutic drugs clinically.

Therefore, there is still a clinical need for the development of pharmaceutically active substances that can be used to inhibit RNA/picornaviruses including coronavirus and enterovirus 71.

## SUMMARY

In one aspect, the present disclosure provides a crystal of a compound of formula (I),

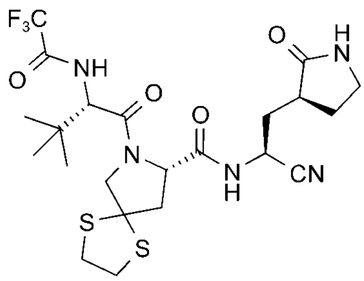

formula (I).

In another aspect, the present disclosure provides crystal form A of the compound of formula (I).

The X-ray powder diffraction pattern expressed in terms of diffraction angles 2$\theta$ of the crystal form A has diffraction peaks at 10.88±0.20°, 15.09±0.20°, 17.67±0.20°, 18.28±0.20°, and 20.64±0.20°.

In yet another aspect, the present disclosure provides a method for preparing a crystal form A of the compound of formula (I), comprising:

mixing the compound of formula (I) with a solvent (i) and a solvent (ii), crystallizing, and separating out a solid, wherein the solvent (i) is selected from at least one of isopropyl acetate, ethyl acetate, and isopropanol, and the solvent (ii) is selected from at least one of *n*-hexane and *n*-heptane.

In another aspect, the present disclosure provides a solvate of the compound of formula (I).

The solvate is selected from a methyl *tert*-butyl ether solvate and a 2-methyltetrahydrofuran solvate. In still another aspect, the present disclosure provides a crystal form B of a methyl *tert*-butyl ether solvate of the compound of

formula (I).

The X-ray powder diffraction pattern expressed in terms of diffraction angles 2$\theta$ of the crystal form B has diffraction peaks at 6.26$\pm$0.20°, 17.66$\pm$0.20°, and 20.24$\pm$0.20°.

In yet another aspect, the present disclosure provides a method for preparing the crystal form B, comprising: mixing the compound of formula (I) with methyl *tert*-butyl ether, and separating out a solid after stirring.

In another aspect, the present disclosure provides a crystal form C of a 2-methyltetrahydrofuran solvate of the compound of formula (I).

The X-ray powder diffraction pattern expressed in terms of diffraction angles 2$\theta$ of the crystal form C has diffraction peaks at 6.21$\pm$0.20°, 17.48$\pm$0.20°, and 20.86$\pm$0.20°.

In still another aspect, the present disclosure provides a method for preparing the crystal form C, comprising: mixing the compound of formula (I) with 2-methyltetrahydrofuran, stirring, and separating out a solid after crystallizing.

In yet another aspect, the present disclosure provides a pharmaceutical combination comprising the crystal form A, the crystal form B, or the crystal form C of the compound of formula (I) described herein, or a combination thereof, and an additional antiviral drug.

In another aspect, the present disclosure provides a pharmaceutical composition comprising the crystal form A, the crystal form B, or the crystal form C of the compound of formula (I) described herein, or a combination thereof, and a pharmaceutically acceptable excipient.

In still another aspect, the present disclosure provides use of the crystal form A, the crystal form B, or the crystal form C of the compound of formula (I) described herein, or a combination thereof, the pharmaceutical combination described herein, or the pharmaceutical composition described herein in the preparation of a medicament for preventing or treating a related disease caused by infection with a coronavirus and/or a picornavirus.

## BRIEF DESCRIPTION OF THE DRAWINGS

FIG. 1 shows an XRPD pattern of a crystal form C of a 2-methyltetrahydrofuran solvate of a compound of formula (I).

FIG. 2 shows a DSC pattern of a crystal form C of a 2-methyltetrahydrofuran solvate of a compound of formula (I).

FIG. 3 shows an XRPD pattern of a crystal form B of a methyl *tert*-butyl ether solvate of a compound of formula (I).

FIG. 4 shows a DSC pattern of a crystal form B of a methyl *tert*-butyl ether solvate of a compound of formula (I).

FIG. 5 shows an XRPD pattern of a crystal form A of the compound of formula (I).

FIG. 6 shows a DSC pattern of a crystal form A of the compound of formula (I).

FIG. 7 shows a TGA pattern of a crystal form A of the compound of formula (I).

FIG. 8 shows the inhibitory effects of a compound of formula (I) on the viral titer in the lungs of mice 2 days (panel A) and 4 days (panel B) after infection in Test Example 4.

FIG. 9 shows the changes in body weight of mice in Test Example 4.

FIG. 10 shows the inhibitory effect of a compound of formula (I) on the viral titer in the brains of mice 4 days after infection in Test Example 4.

## DETAILED DESCRIPTION

As a novel 3CL protease inhibitor, the compound of formula (I) has broad-spectrum anti-coronavirus activity, and the compound of formula (I) has the following structure:

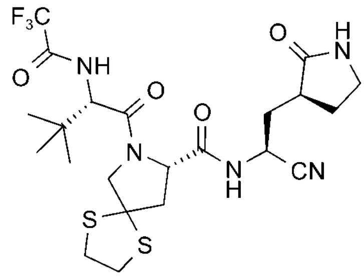

formula (I).

The present disclosure provides a crystal of a compound of formula (I).

The present disclosure provides crystal form A of the compound of formula (I), wherein the X-ray powder diffraction pattern expressed in terms of diffraction angles 2$\theta$ of the crystal form A has diffraction peaks at 10.88±0.20°, 15.09±0.20°, 17.67±0.20°, 18.28±0.20°, and 20.64±0.20°.

In some embodiments, the X-ray powder diffraction pattern expressed in terms of diffraction angles 2$\theta$ of the crystal form A of the compound of formula (I) has diffraction peaks at 10.88±0.20°, 15.09±0.20°, 16.61±0.20°, 17.67 ±0.20°, 18.28±0.20°, 18.50±0.20°, 20.09±0.20°, and 20.64±0.20°.

In some embodiments, the X-ray powder diffraction pattern expressed in terms of diffraction angles 2$\theta$ of the crystal form A of the compound of formula (I) has diffraction peaks at 10.27±0.20°, 10.88±0.20°, 11.86±0.20°, 15.09 ±0.20°, 16.61±0.20°, 17.67±0.20°, 18.28±0.20°, 18.50±0.20°, 20.09±0.20°, and 20.64±0.20°.

In some embodiments, the X-ray powder diffraction pattern expressed in terms of diffraction angles 2$\theta$ of the crystal form A of the compound of formula (I) has diffraction peaks at 9.29±0.20°, 10.27±0.20°, 10.88±0.20°, 10.97 ±0.20°, 11.86±0.20°, 14.27±0.20°, 14.92±0.20°, 15.09±0.20°, 15.61±0.20°, 15.78±0.20°, 16.61±0.20°, 17.67 ±0.20°, 18.28±0.20°, 18.50±0.20°, 20.09±0.20°, 20.64±0.20°, 22.18±0.20°, 23.80±0.20°, and 25.63±0.20°.

In some embodiments, the X-ray powder diffraction pattern expressed in terms of diffraction angles 2$\theta$ of the crystal form A of the compound of formula (I) has diffraction peaks as shown in Table 3.

In some embodiments, the crystal form A of the compound of formula (I) has the X-ray powder diffraction pattern expressed in terms of diffraction angles 2$\theta$ substantially as shown in FIG. 5.

In some embodiments, the crystal form A of the compound of formula (I) has a DSC pattern with a peak at 213.78 °C ± 5.0 °C.

In some embodiments, the crystal form A of the compound of formula (I) has a DSC pattern substantially as shown in FIG. 6.

The present disclosure further provides a method for preparing a crystal form A of the compound of formula (I), comprising:
mixing the compound of formula (I) with a solvent (i) and a solvent (ii), crystallizing, and separating out a solid, wherein the solvent (i) is selected from at least one of isopropyl acetate, ethyl acetate, and isopropanol, preferably isopropyl acetate, and the solvent (ii) is selected from at least one of *n*-hexane and n-heptane, preferably *n*-heptane.

The above mixing of the compound of formula (I) with the solvent (i) and the solvent (ii) comprises at least the following specific modes: mixing the compound of formula (I) with the solvent (i) and then further mixing with the solvent (ii); or mixing the compound of formula (I) with a mixed solvent of the solvent (i) and the solvent (ii).

In some embodiments, in the preparation method for the crystal form A, the volume (mL) of the solvent (i) is 1-100 times, preferably 2-50 times the mass (g) of the compound.

In some embodiments, in the preparation method for the crystal form A, the volume (mL) of the solvent (ii) is 2-100 times, preferably 5-50 times the mass (g) of the compound.

In some embodiments, the crystallization in the preparation method for the crystal form A is performed at 10-80 °C, preferably at 15-45 °C, and more preferably at 20-30 °C.

In some embodiments, in the preparation method for the crystal form A, the separation step comprises filtering, collecting the solid, and drying.

The present disclosure further provides a solvate of the compound of formula (I), and solvate is selected from a methyl *tert*-butyl ether solvate and a 2-methyltetrahydrofuran solvate.

In some embodiments, in the solvate of the compound of formula (I), the molar ratio of the compound of formula (I) to the solvent is about 0.5-2.

In some embodiments, in the solvate of the compound of formula (I), the molar ratio of the compound of formula (I) to the solvent is about 0.8-1.2.

In some embodiments, in the solvate of the compound of formula (I), the molar ratio of the compound of formula (I)

to the solvent is about 1.0.

The present disclosure further provides crystal form B of a methyl *tert*-butyl ether solvate of the compound of formula (I), wherein the X-ray powder diffraction pattern expressed in terms of diffraction angles 2$\theta$ of the crystal form B has diffraction peaks at 6.26±0.20°, 17.66±0.20°, and 20.24±0.20°.

In some embodiments, the X-ray powder diffraction pattern expressed in terms of diffraction angles 2$\theta$ of the crystal form B has diffraction peaks at 6.26±0.20°, 7.27±0.20°, 11.47±0.20°, 13.06±0.20°, 15.57±0.20°, 17.66±0.20°, 20.24±0.20°, and 22.72±0.20°.

In some embodiments, the X-ray powder diffraction pattern expressed in terms of diffraction angles 2$\theta$ of the crystal form B has diffraction peaks at 6.26±0.20°, 7.27±0.20°, 8.84±0.20°, 10.18±0.20°, 10.30±0.20°, 11.47±0.20°, 13.06±0.20°, 14.44±0.20°, 15.57±0.20°, 17.66±0.20°, 20.24±0.20°, 20.51±0.20°, and 22.72±0.20°.

In some embodiments, the X-ray powder diffraction pattern expressed in terms of diffraction angles 2$\theta$ of the crystal form B has diffraction peaks as shown in Table 2.

In some embodiments, the crystal form B has the X-ray powder diffraction pattern expressed in terms of diffraction angles 2$\theta$ substantially as shown in FIG. 3.

In some embodiments, the crystal form B has a DSC pattern with peaks at 93.36±5.0 °C, 170.25±5.0 °C, and 214.18±5.0 °C.

In some embodiments, the crystal form B has a DSC pattern substantially as shown in FIG. 4.

The present disclosure further provides a method for preparing a crystal form B, comprising:
mixing the compound of formula (I) with methyl *tert*-butyl ether, and separating out a solid after stirring.

In some embodiments, in the preparation method for the crystal form B, the volume (mL) of the methyl *tert*-butyl ether is 2-100 times, preferably 5-50 times, and more preferably 10-30 times the mass (g) of the compound of formula (I).

In some embodiments, in the preparation method for the crystal form B, the stirring step is performed at *15-55* °C, preferably at 45-55 °C, and more preferably at 50-55 °C.

In some embodiments, in the preparation method for the crystal form B, the compound of formula (I) is a 2-methyltetrahydrofuran solvate of the compound of formula (I).

The present disclosure further provides crystal form C of a 2-methyltetrahydrofuran solvate of the compound of formula (I), wherein the X-ray powder diffraction pattern expressed in terms of diffraction angles 2$\theta$ of the crystal form C has diffraction peaks at 6.21±0.20°, 17.48±0.20°, and 20.86±0.20°.

In some embodiments, the X-ray powder diffraction pattern expressed in terms of diffraction angles 2$\theta$ of the crystal form C has diffraction peaks at 6.21±0.20°, 7.04±0.20°, 11.71±0.20°, 17.48±0.20°, 20.59±0.20°, and 20.86 ±0.20°.

In some embodiments, the X-ray powder diffraction pattern expressed in terms of diffraction angles 2$\theta$ of the crystal form C has diffraction peaks at 6.21±0.20°, 7.04±0.20°, 11.71±0.20°, 14.74±0.20°, 15.77±0.20°, 17.48±0.20°, 20.59±0.20°, and 20.86±0.20°.

In some embodiments, the X-ray powder diffraction pattern expressed in terms of diffraction angles 2$\theta$ of the crystal form C has diffraction peaks as shown in Table 1.

In some embodiments, the crystal form C has the X-ray powder diffraction pattern expressed in terms of diffraction angles 2$\theta$ substantially as shown in FIG. 1.

In some embodiments, the crystal form C has a DSC pattern with peaks at 90.39±5.0 °C, 167.59±5.0 °C, and 214.22±5.0 °C.

In some embodiments, the crystal form C has a DSC pattern substantially as shown in FIG. 2.

The present disclosure further provides a method for preparing a crystal form C, comprising:
mixing the compound of formula (I) with 2-methyltetrahydrofuran, stirring, and separating out a solid after crystallizing.

In some embodiments, in the preparation method for the crystal form C, the volume (mL) of the 2-methyltetrahydrofuran is 2-50 times, preferably 3-20 times, and more preferably 5-15 times the mass (g) of the compound of formula (I).

In some embodiments, in the preparation method for the crystal form C, the stirring step is performed at 15-80 °C, preferably 40-80 °C, for example, 50-60 °C or 70-80 °C.

In some embodiments, in the preparation method for the crystal form C, the crystallization is cooling crystallization.

In some embodiments, in the preparation method for the crystal form C, the cooling step is cooling the reaction system to 0-35 °C, preferably to 5-30 °C, and more preferably to 10-25 °C.

In another aspect, the present disclosure further provides a pharmaceutical combination comprising the crystal form A, the crystal form B, or the crystal form C of the compound of formula (I) described above, or a combination thereof, and an additional antiviral drug.

In another aspect, the present disclosure provides a pharmaceutical composition comprising the crystal form A, the crystal form B, or the crystal form C of the compound of formula (I) described above, or a combination thereof, and a pharmaceutically acceptable excipient; optionally, the pharmaceutical composition further comprises an additional

antiviral drug.

In some embodiments, the additional antiviral drug is ritonavir.

In another aspect, the present disclosure provides use of the crystal form A, the crystal form B, or the crystal form C of the compound of formula (I) described above, or a combination thereof, the pharmaceutical combination described above, or the pharmaceutical composition described above in the preparation of a medicament for preventing or treating a related disease caused by infection with a coronavirus and/or a picornavirus.

In another aspect, the present disclosure provides use of the crystal form A, the crystal form B, or the crystal form C of the compound of formula (I) described above, or a combination thereof, the pharmaceutical combination described above, or the pharmaceutical composition described above in the prevention or treatment of a related disease caused by infection with a coronavirus and/or a picornavirus.

In another aspect, the present disclosure provides the crystal form A, the crystal form B, or the crystal form C of the compound of formula (I) described above, or a combination thereof, the pharmaceutical combination described above, or the pharmaceutical composition described above for preventing or treating a related disease caused by infection with a coronavirus and/or a picornavirus.

In another aspect, the present disclosure provides a method for treating a related disease caused by infection with a coronavirus and/or a picornavirus, comprising administering to a subject (e.g., a mammal, preferably a human) in need of such treatment a therapeutically effective amount of the crystal form A, the crystal form B, or the crystal form C of the compound of formula (I) described above, or a combination thereof, or the pharmaceutical combination described above, or the pharmaceutical composition described above.

The related disease caused by infection with the coronavirus and/or the picornavirus described herein includes, but is not limited to, respiratory tract infection, pneumonia, or a complication thereof.

The coronavirus described herein is selected from SARS-CoV, MERS-CoV, H229E-CoV, HKU1-CoV, NL63-CoV, OC43-CoV, and SARS-CoV-2. The picornavirus described herein is selected from enterovirus type 71.

The crystal form A, crystal form B, or crystal form C of the compound of formula (I) described herein has at least one advantage in terms of pharmacological effect, physicochemical property, and the like, and is suitable for preparing a desired pharmaceutical composition.

## TERMINOLOGY AND DEFINITIONS

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be understood according to its common meaning in the field. When referring to a trade name, it is intended to refer to its corresponding commercial product or its active ingredient.

The term "solvate" refers to a complex or aggregate formed by one or more molecules of a solute and one or more molecules of a solvent. Solvates typically have a substantially fixed molar ratio of solute and solvent. The term also includes cage-like compounds, including cage-like compounds with water. Representative solvents include, for example, water, methanol, ethanol, isopropanol, acetic acid, 2-methyltetrahydrofuran, methyl *tert*-butyl ether, and the like. When the solvent is water, the solvate formed is a hydrate.

The term "about" is used in the present disclosure to mean approximately, around, roughly, or approximately. When the term "about" is used in conjunction with a numerical range, the range is modified by extending the upper and lower limits of the stated numerical range. Unless otherwise stated, the term "about" is used herein to modify the upper and lower limits of the stated value by a numerical value that deviates by 10%.

Unless otherwise stated, the term "comprise" and variations thereof such as "comprises" or "comprising" should be understood in an open, non-exclusive sense, i.e., "including but not limited to".

"Optional embodiment" or "embodiment" mentioned in the disclosure means that a specific reference element, structure, or feature described in connection with the embodiment is included in at least one embodiment. Thus, the appearances of the phrases "optional embodiment" or "embodiment" in various places throughout this disclosure are not necessarily all referring to the same embodiment. Furthermore, the particular elements, structures, or features may be combined in any suitable manner in one or more embodiments.

The room temperature described herein refers to 20$\pm$5.0 °C.

The range "m-n" described in the present disclosure represents an abbreviated representation of any combination of real numbers between m and n, where m and n are both real numbers.

The "X-ray powder diffraction pattern" described herein is obtained by measurement using CuK$\alpha$ radiation.

The "X-ray powder diffraction pattern" or "XRPD pattern" described herein refers to a set of X-ray powder diffraction peaks obtained according to the Bragg's equation 2d Sin $\theta$ = n$\lambda$ (where d is the interplanar spacing, $\theta$ is the diffraction angle, $\lambda$ is the wavelength of the incident X-ray, the diffraction order n is any positive integer, and the first-order

diffraction peak is generally taken, i.e., n = 1). When X-ray is incident on a certain atomic plane with the d-lattice plane spacing of a crystal or a partial crystal sample at a grazing angle $\theta$ (the complementary angle of the incident angle, also known as Bragg angle), the Bragg's equation can be satisfied.

For the same crystal forms of the same compound, the peak positions in the XRPD patterns are similar on the whole, and the relative intensity error may be large. It should also be noted that in the identification of mixtures, some diffraction lines may be absent due to factors such as content reduction, and in this case, it is not necessary to rely on all diffraction peaks observed in a high-purity sample, and even one diffraction peak may be characteristic for a given crystal.

As used herein, "2 $\theta$ or 2 $\theta$ angle" refers to a diffraction angle; $\theta$ is the Bragg angle in ° or degrees.

To those skilled in the art, due to factors such as crystal defects and measurement errors, the molar ratio of the compound of the present disclosure to the acid/base molecules and the solvent molecules in the solvate often has certain degrees of errors. Generally, $\pm$10% falls within a reasonable error range. The error varies to some extent depending on the context in which it is used, and the variation is no more than $\pm$10%, preferably $\pm$5%.

The term "therapeutically effective amount" refers to an amount of the compound of the present disclosure for (i) treating a specific disease, condition or disorder; (ii) alleviating, ameliorating or eliminating one or more symptoms of a specific disease, condition or disorder, or (iii) delaying the onset of the one or more symptoms of the specific disease, condition or disorder described herein. The amount of the compound of the present disclosure constituting the "therapeutically effective amount" varies depending on the compound, the disease state and its severity, the administration regimen, and the age of the mammal to be treated, but can be determined routinely by those skilled in the art in accordance with their knowledge and the present disclosure.

The term "treating" or "treatment" means administering the compound or formulation described herein to ameliorate or eliminate a disease or one or more symptoms associated with the disease, and includes:

(i) inhibiting a disease or a disease state, i.e., arresting its development; and
(ii) alleviating a disease or a disease state, i.e., causing its regression.

The term "prevent", "preventing", or "prevention" means administering the compound or formulation described herein to prevent a disease or one or more symptoms associated with the disease, and includes:
preventing the occurrence of the disease or disease state in a subject (e.g., a mammal), particularly when such a subject is predisposed to the disease state but has not yet been diagnosed with it.

In the present application, the term "subject" or "patient" includes mammals and non-mammals. Examples of mammals include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, guinea pigs, and the like. Examples of non-human mammals include, but are not limited to, birds, fish, and the like.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.

The term "pharmaceutical combination" refers to a combination of two or more active ingredients. In some embodiments of the present disclosure, the active ingredients in the pharmaceutical combination may be administered simultaneously, and in some embodiments of the present disclosure, the active ingredients in the pharmaceutical combination may also be administered separately or sequentially.

The pharmaceutical composition of the present disclosure can be prepared by combining the compound of the present disclosure or a pharmaceutically acceptable salt thereof or a solvate thereof with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the solvate thereof, or the pharmaceutical composition comprising the compound or the pharmaceutically acceptable salt thereof or the solvate thereof disclosed herein include, but are not limited to, oral, rectal, local, inhalation, parenteral, sublingual, intravaginal, intranasal, intraocular, intraperitoneal, intramuscular, subcutaneous and intravenous administration.

The pharmaceutical composition of the present disclosure can be manufactured by methods well known in the art, such as conventional methods of mixing, dissolving, granulating, emulsifying, lyophilizing, and the like.

In some embodiments, the pharmaceutical composition is in an oral form. For oral administration, the pharmaceutical composition can be formulated by mixing the active compounds with pharmaceutically acceptable excipients well known in the art. These excipients enable the compounds of the present disclosure or a pharmaceutically acceptable salt thereof or a solvate thereof to be formulated into tablets, pills, lozenges, dragees, capsules, liquids, gels, slurries, suspensions, and the like, for oral administration to patients.

A solid oral composition can be prepared by conventional mixing, filling, or tableting. For example, it can be obtained by the following method: mixing the active compounds with solid excipients, optionally grinding the resulting mixture, adding additional suitable excipients if desired, and processing the mixture into granules to get the core parts of tablets or dragees.

The pharmaceutical compositions may also be suitable for parenteral administration, such as sterile solutions, suspensions, or lyophilized products in suitable unit dosage forms.

The therapeutically effective amount of the crystal form of the compound of formula (I) or the solvate thereof contained in the pharmaceutical composition of the present disclosure is selected from 0.001 mg/kg body weight to 1000 mg/kg body weight, e.g., 0.01 mg/kg body weight to 500 mg/kg body weight, in individual or separated doses.

One skilled in the art recognizes that measured data of XRPD peak positions and/or intensities for a given crystal form of the same compound will vary within a margin of error. The $2\theta$ values in the present disclosure encompass appropriate error ranges, which are generally represented by "±". For example, a $2\theta$ value represented by a specific angle value±0.20° in the present disclosure means that the specific angle value has an error floating range of ±0.20°, that is, 5.92±0.20° $2\theta$ means that $2\theta$ is within the range of 6.12 to 5.72. Depending on the sample preparation technique, the calibration technique applied to the instrument, human operating deviations, and the like, those skilled in the art recognize that an appropriate error range for the XRPD diffraction angles may be ±0.20°, ±0.15°, ±0.10°, ±0.05°, or less, with some variability permitted for the peak intensities. The term "substantially the same" or "substantially as shown in ..." when used to describe an XRPD pattern refers to a pattern including diffraction peaks having at least 50%, at least 60%, at least 70%, at least 80%, at least 90%, at least 95%, or at least 99% of the diffraction angles within a standard deviation range of ±0.2° $2\theta$.

As those skilled in the art will recognize, measurement data for a DSC pattern of a given crystal form of the same compound will vary within a margin of error. A single peak value (expressed in degrees Celsius) allows for an appropriate error range. Generally, the error range is represented by "±". For the same crystal form of the same compound, the thermal transition temperature and melting point errors in successive analyses are typically within ±5.0 °C. For example, a peak of "170.25±5.0" means within the range of 165.25 to 175.25. Depending on sample preparation technique, the calibration technique applied to the instrument, human operating variations, and the like, those skilled in the art recognize that appropriate error ranges for single peak values may be ±5.0, ±4.0, ±3.0, ±2.0, or smaller.

The salt forms and/or crystal forms of the present disclosure may also be isotopically labeled. The present disclosure also includes isotopically labeled compounds of the present disclosure which are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Certain isotopically labeled compounds of the present disclosure (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$, and $^{18}F$, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., $^{2}H$) may provide certain therapeutic advantages (e.g., increased in vivo half-life or reduced dosage) resulting from greater metabolic stability and thus may be preferred in certain circumstances in which deuterium substitution may be partial or complete, wherein partial deuterium substitution refers to substitution of at least one hydrogen with deuterium.

The compounds disclosed herein or a pharmaceutically acceptable salt thereof or a solvate thereof can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples disclosed herein.

The chemical reactions of the specific embodiments disclosed herein are conducted in a suitable solvent that must be suitable for the chemical changes in the present disclosure and the reagents and materials required. In order to obtain the compounds disclosed herein or a pharmaceutically acceptable salt thereof or a solvate thereof, it is sometimes necessary for those skilled in the art to modify or select a synthesis procedure or a reaction process based on the existing embodiments.

Test conditions for the instruments used in the experiments of the present disclosure:

1. X-ray powder diffraction

Instrument model: Bruker D8 Focus
X-ray light source: Cu Kα
Kα1 (Å): 1.54060; Kα2 (Å): 1.54439; Kα2/Kα1 intensity ratio: 0.50
Wavelength λ (Å): 1.54060
Slit (°): 2.5
Scan mode: θ/2θ, scan range: 3-40° (2$\theta$ angle)
Retention time (s): 0.12
Scanning step length (° 2$\theta$): 0.01
Scanning flow rate: 5°/min
Voltage: 40 kV
Current: 40 mA

2. Differential scanning calorimeter

Instrument model: Discovery DSC2500
Purging gas: nitrogen
Sample tray: aluminum tray, non-sealing gland
Method: linear heating
Ramping rate: 10 °C/min
Temperature range: 30 °C to 300 °C; or 30 °C to 400 °C;

3. Thermogravimetric analyzer

Instrument model: Discovery TA 55
Purging gas: nitrogen
Sample tray: platinum, open
Method: linear heating
Ramping rate: 10 °C/min
Temperature range: 30 °C to 300 °C

4. Dynamic vapor sorption instrument

Instrument model: DVS Intrinsic PLUS
DVS parameters:

Temperature: 25 °C;
Balancing: dm/dt = 0.002%/min
RH (%) test gradient: 10%
Range of RH (%) test gradient: 0%-90%-0%.

The compounds disclosed herein can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples disclosed herein.

The chemical reactions of the specific embodiments disclosed herein are conducted in a suitable solvent that must be suitable for the chemical changes in the present disclosure and the reagents and materials required. In order to obtain the compounds disclosed herein, it is sometimes necessary for those skilled in the art to modify or select a synthesis procedure or a reaction process based on the existing embodiments.

## DETAILED DESCRIPTION

The present disclosure is described in detail below by way of examples, which, however, are not intended to disadvantageously limit the scope of the present disclosure in any way. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure. All reagents used in the present disclosure are commercially

available and can be used without further purification.

Unless otherwise stated, the ratios expressed for mixed solvents are volume mixing ratios. Unless otherwise stated, % refers to wt%.

Compounds are named either manually or by ChemDraw® software, and supplier's catalog names are given for commercially available compounds.

The structures of the compounds are determined by nuclear magnetic resonance (NMR) and/or mass spectrometry (MS). NMR shifts are given in $10^{-6}$ (ppm). The solvents for NMR determination are deuterated dimethyl sulfoxide, deuterated chloroform, deuterated methanol, and the like, and the internal standard is tetramethylsilane (TMS).

## Example 1: Preparation of Compound of Formula (I)

1.1. Preparation of compound 1-1:

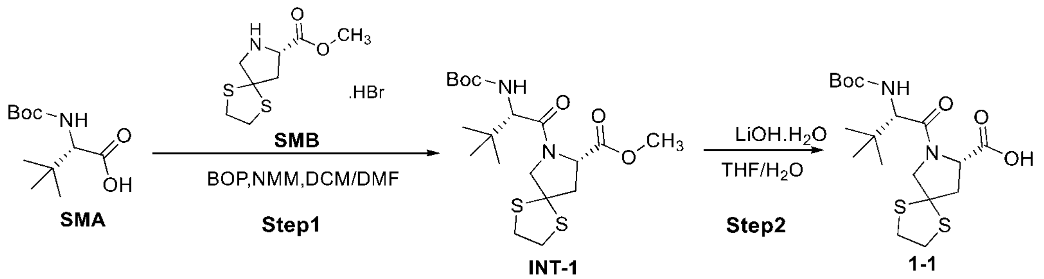


Step 1: Starting material SMA (2.74 g, 11.85 mmol), 35 mL of dichloromethane, and 35 mL of DMF were added to a reaction flask. The mixture was cooled to 0 °C. Starting material SMB (3.56 g, 11.86 mmol), benzotriazol-1-yloxytris(dimethylamino)phosphonium hexafluorophosphate (BOP, 6.29 g, 14.22 mmol), and *N*-methylmorpholine (NMM, 3.91 mL, 35.56 mmol) were sequentially added. The mixture was heated to room temperature and allowed to react for 10 h. After the reaction was completed, a proper amount of dichloromethane was added. The organic phase was washed sequentially with a 1 N aqueous hydrochloric acid solution and saturated brine. After the washing was completed, the organic phase was dried over anhydrous sodium sulfate and concentrated to dryness, and the residue was subjected to column chromatography to give 3.71 g of INT-1; ESI-MS: 433.2 m/z $[M+H]^+$; $^1H$ NMR (400 MHz, DMSO-$d_6$): $\delta_H$: 6.75 (d, J = 9.2 Hz, 1H), 4.38 (t, J = 8.2 Hz, 1H), 4.25 (d, J = 10.9 Hz, 1H), 4.11 (d, J = 9.3 Hz, 1H), 3.93 (t, J = 9.3 Hz, 1H), 3.62 (s, 3H), 3.40-3.31 (m, 4H), 2.70 (dd, J = 13.1, 7.9 Hz, 1H), 2.37 (dd, J = 13.2, 8.4 Hz, 1H), 1.37 (s, 9H), 0.94 (s, 9H).

Step 2: INT-1 (3.71 g, 8.58 mmol), 37 mL of THF, 37 mL of purified water, and lithium hydroxide monohydrate (0.72 g, 17.16 mmol) were added to a reaction flask, and the mixture was allowed to react at room temperature for 2 h. After the reaction was completed, the mixture was adjusted to pH 4 with concentrated hydrochloric acid and filtered to give 3.4 g of compound 1-1; ESI-MS: 419.2 m/z $[M+H]^+$; $^1H$ NMR (400 MHz, DMSO-$d_6$): $\delta_H$:12.68 (s, 1H), 6.71 (d, J = 9.4 Hz, 1H), 4.38-4.19 (m, 2H), 4.11 (d, J = 9.4 Hz, 1H), 3.88 (d, J = 10.9 Hz, 1H), 3.41-3.29(m, 4H), 2.69 (dd, J = 13.1, 7.9 Hz, 1H), 2.34 (dd, J = 13.2, 8.9 Hz, 1H), 1.38 (s, 9H), 0.94 (s, 9H).

1.2. Preparation of compound 1-2:

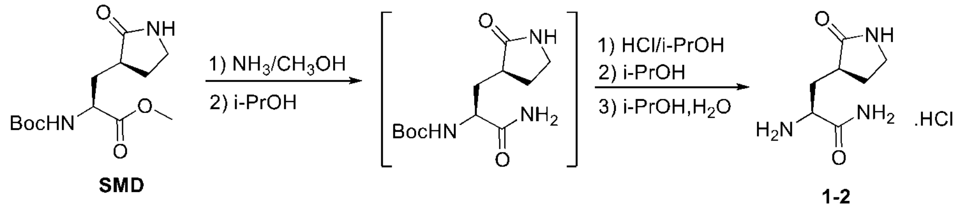


A solution of ammonia in methanol (700 mL, 7 mol/L) and starting material SMD (100 g, 0.349 mol) were added to a reaction flask and stirred until the solid was completely dissolved. The mixture was allowed to react for 36 h with the temperature maintained at 25±5 °C. After the reaction was completed, the reaction mixture was concentrated until the residual reaction mixture was about 250 mL, then 300 mL of isopropanol was added, and concentration under reduced

pressure was continued until the residual reaction mixture was about 250 mL (this procedure was repeated three times). The system was purged with nitrogen and cooled to 10±5 °C. 500 mL of a solution of hydrogen chloride in isopropanol (4 mol/L) was added to the reaction kettle, and after the addition was completed, the mixture was heated to 25±5 °C and allowed to react for 9 h with the temperature maintained at 25±5 °C. After the reaction was completed, the reaction mixture was concentrated under reduced pressure until the residual volume of the reaction mixture was about 250 mL, then 300 mL of isopropanol was added, and concentration under reduced pressure was continued until the residual volume of the reaction mixture was about 250 mL (this procedure was repeated twice). Then, 100 mL of isopropanol was added, and the mixture was stirred for 30±5 min and filtered. The filter cake was rinsed with 50 mL of isopropanol to give a wet product, which was then dried under vacuum at 45±5 °C to give 66.7 g of compound 1-2; $^1$H NMR (400 MHz, DMSO-$d_6$): $\delta_H$:8.45 (d, J = 5.1 Hz, 3H), 8.25-8.04 (m, 1H), 7.95 (s, 1H), 7.67-7.49 (m, 1H), 3.85-3.80 (m, 1H), 3.19-3.13 (m, 2H), 2.59-2.51 (m, 1H), 2.32-2.27 (m, 1H), 2.05-1.98 (m, 1H), 1.82-1.66 (m, 2H); ESI-MS: 172.1 m/z [M+H]$^+$. 1.3. Preparation of compound of formula (I):

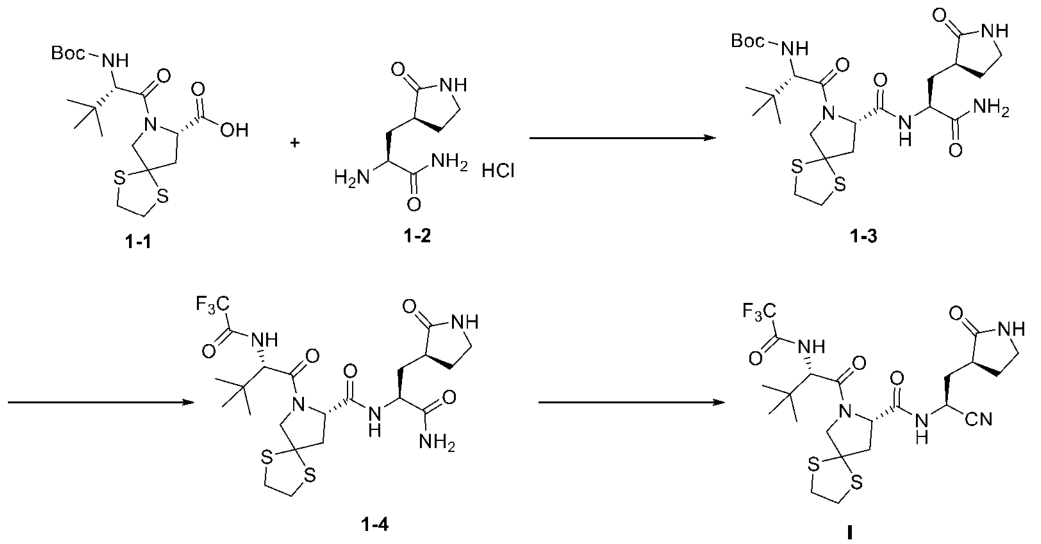

1-1 1-2 1-3

1-4 I

Step 1: Compound 1-1 (419 mg, 1 mmol) was placed in a double-necked flask, 5 mL of dichloromethane was added under nitrogen atmosphere, then *O*-(7-azabenzotriazol-1-yl)-*N,N,N',N'*-tetramethyluronium hexafluorophosphate (400 mg, 1.1 mmol) was added, and the reaction mixture was stirred at room temperature for 1 h. Compound 1-2 (1 mmol) was dissolved in 1 mL of dichloromethane, and the mixture was added to the above system. Subsequently, *N,N*-diisopropylethylamine (2 mmol) was added under an ice-water bath. The ice-water bath was then removed, and the system was stirred at room temperature overnight. For post-treatment: 50 mL of dichloromethane was added, and the mixture was washed three times with a 1 M aqueous hydrochloric acid solution and then washed three times with a saturated aqueous sodium bicarbonate solution. The organic phase was washed with saturated brine, dried over anhydrous sodium sulfate, filtered, and concentrated to dryness by rotary evaporation to give compound 1-3 (469 mg). ESI-MS: m/z 572.3[M+H]$^+$.

Step 2: Compound 1-3 (572 mg, 1 mmol) was dissolved in 3 mL of a 4 M solution of hydrogen chloride in 1,4-dioxane, and the mixture was stirred at ambient temperature. After the starting materials were substantially consumed as detected by thin layer chromatography, the mixture was concentrated to dryness by rotary evaporation to remove the solvent. The resulting crude product was dissolved in 2 mL of dichloromethane, and triethylamine (3 mmol) was added under nitrogen atmosphere. The system was placed in an ice-water bath, and trifluoroacetic anhydride (1.2 mmol) was added dropwise. After the starting materials were substantially consumed as detected by thin layer chromatography, 50 mL of dichloromethane was added, and the mixture was washed three times with a 1 M aqueous hydrochloric acid solution and then washed three times with a saturated aqueous sodium bicarbonate solution. The organic phase was washed with saturated brine, dried over anhydrous sodium sulfate, and subjected to column chromatography to give compound 1-4 (265 mg). ESI-MS: m/z 568.3[M+H]$^+$.

Step 3: Compound 1-4 (113 mg, 0.2 mmol) and Burgess reagent (1.5 eq) were added to a two-necked flask. The system was purged with nitrogen three times, and then dichloromethane dried with molecular sieves was added. The mixture was stirred at room temperature overnight, and the starting materials were substantially consumed as detected by thin layer chromatography. For post-treatment: The mixture was subjected to column chromatography to give a **compound of formula (I)** (41 mg), which was confirmed in an amorphous form by XRPD. $^1$H NMR (400 MHz, DMSO-$d_6$) δ 9.46 (d, *J* = 8.7 Hz, 1H), 9.05 (d, *J* = 8.6 Hz, 1H), 7.67 (s, 1H), 4.97 (ddd, *J* = 11.0, 8.5, 5.0 Hz, 1H), 4.53 (d, *J* = 8.7 Hz, 1H), 4.34 (dd, *J* = 9.9, 7.1 Hz, 1H), 4.26-4.14 (m, 1H), 3.92 (d, *J* = 10.9 Hz, 1H), 3.50-3.34 (m, 4H), 3.22-3.11 (m, 1H), 3.06 (td, *J* =

9.3, 7.1 Hz, 1H), 2.68-2.58 (m, 1H), 2.50-2.43 (m, 1H), 2.31 (dd, *J* = 13.0, 10.0 Hz, 1H), 2.23-2.07 (m, 2H), 1.71 (tdd, *J* = 14.9, 10.3, 7.4 Hz, 2H), 0.99 (s, 9H). ESI-MS: 550.3 m/z $[M+H]^+$.

**Example 2: Preparation of Crystal Form C of 2-Methyltetrahydrofuran Solvate of Compound of Formula (I)**

Compound 1-4 (10.00 g) was dissolved in 100 mL of dichloromethane, and 8.39 g of Burgess reagent was added. The mixture was allowed to react for 1 h with the temperature maintained at 25-26 °C. After the reaction was completed, the mixture was quenched with 50 mL of a saturated aqueous sodium bicarbonate solution. The organic phase was separately washed with 50 mL of a 1 M aqueous hydrochloric acid solution and 50 mL of a saturated aqueous sodium chloride solution, and concentrated under reduced pressure to give the compound of formula (I). The obtained compound of formula (I) was added to 100 mL of 2-methyltetrahydrofuran, heated to 70-80 °C, and completely dissolved. The mixture was slowly cooled for crystallization and filtered to give a solid (7.92 g). The obtained solid was confirmed to be the crystal form C of the 2-methyltetrahydrofuran solvate of the compound of formula (I) by methods such as nuclear magnetic resonance, TGA, and X-ray powder diffraction. The XRPD pattern of the crystal form C is shown in FIG. 1, the DSC pattern thereof is shown in FIG. 2, and the positions of XRPD diffraction peaks are shown in Table 1 below.

Table 1

| Peak No. | $2\theta$ value [° or degree] | d [Å] | Relative intensity (%) |
|---|---|---|---|
| 1 | 6.21 | 14.22 | 25 |
| 2 | 7.04 | 12.55 | 13 |
| 3 | 8.70 | 10.15 | 6 |
| 4 | 11.71 | 7.55 | 16 |
| 5 | 12.42 | 7.12 | 8 |
| 6 | 14.13 | 6.27 | 5 |
| 7 | 14.74 | 6.01 | 15 |
| 8 | 14.98 | 5.91 | 6 |
| 9 | 15.77 | 5.62 | 18 |
| 10 | 17.23 | 5.14 | 12 |
| 11 | 17.48 | 5.07 | 100 |
| 12 | 17.93 | 4.94 | 7 |
| 13 | 20.06 | 4.42 | 8 |
| 14 | 20.59 | 4.31 | 23 |
| 15 | 20.86 | 4.25 | 34 |

$^1H$ NMR(400 MHz, DMSO-$d_6$) $\delta$ 9.47 (d, *J* = 7.6 Hz, 1H), 9.06 (d, *J* = 8.6 Hz, 1H), 7.68 (s, 1H), 4.99-4.93(m, 1H), 4.52 (d, *J* = 7.0 Hz, 1H), 4.32 (dd, *J* = 9.9Hz, 7.1 Hz, 1H), 4.19 (d, *J* = 11.0 Hz, 1H), 3.91 (d, *J* = 10.9 Hz, 1H), 3.79-3.86(m,1H),3.78-3.71(m,1H),3.58-3.51(m,1H),3.47-3.35 (m, 4H), 3.14 (t, *J* = 9.3 Hz, 1H), 3.08-3.02 (m, 1H), 2.61 (dd, *J* = 13.0Hz, 7.1 Hz, 1H), 2.46-2.39 (m, 1H), 2.35-2.25 (m, 1H), 2.18-2.07 (m, 2H), 2.00-1.75(m,3H),1.75-1.67 (m, 2H),1.35-1.27 (m, 1H), 1.12(d, *J*=6.1Hz,3H), 0.97 (s, 9H).

**Example 3: Preparation of Crystal Form B of Methyl *tert*-Butyl Ether Solvate of Compound of Formula (I)**

1.6 kg of the 2-methyltetrahydrofuran solvate of the compound of formula (I) was dissolved in 4.5 L of dichloromethane, and the mixture was concentrated to dryness under reduced pressure to give a solid, which was then crushed and added in batches to 45 L of methyl *tert*-butyl ether. After the addition was completed, the mixture was heated to 50-55 °C, slurried for 1 h, then slowly cooled to 20 °C, filtered, and dried to give a solid (1.41 kg). The obtained solid was confirmed to be the crystal form B of the methyl *tert*-butyl ether solvate of the compound of formula (I) by methods such as nuclear magnetic resonance, TGA, and X-ray powder diffraction. The XRPD pattern of the crystal form B is shown in FIG. 3, the DSC pattern thereof is shown in FIG. 4, and the positions of XRPD diffraction peaks are shown in Table 2 below.

Table 2

| Peak No. | $2\theta$ value [° or degree] | d [Å] | Relative intensity (%) |
|---|---|---|---|
| 1 | 6.26 | 14.11 | 44 |
| 2 | 7.27 | 12.15 | 15 |
| 3 | 8.84 | 10.00 | 5 |
| 4 | 10.18 | 8.68 | 5 |
| 5 | 10.30 | 8.58 | 5 |
| 6 | 11.47 | 7.71 | 11 |
| 7 | 12.00 | 7.37 | 7 |
| 8 | 12.42 | 7.12 | 8 |
| 9 | 13.06 | 6.78 | 14 |
| 10 | 14.44 | 6.13 | 12 |
| 11 | 15.43 | 5.74 | 14 |
| 12 | 15.57 | 5.69 | 19 |
| 13 | 17.66 | 5.02 | 100 |
| 14 | 17.95 | 4.94 | 19 |
| 15 | 20.24 | 4.38 | 41 |
| 16 | 20.33 | 4.36 | 30 |
| 17 | 20.51 | 4.33 | 23 |
| 18 | 22.72 | 3.91 | 23 |

1H NMR(400 MHz, DMSO-$d_6$): δ 9.48 (d, *J* = 8.7 Hz, 1H), 9.06 (d, *J* = 8.6 Hz, 1H), 7.68 (s, 1H), 5.00-4.94 (m, 1H), 4.53 (d, *J* = 8.7 Hz, 1H), 4.33 (dd, *J* = 9.8 Hz, 7.1 Hz, 1H), 4.19 (d, *J* = 10.7 Hz, 1H), 3.92 (d, *J* = 10.9 Hz, 1H), 3.44-3.37 (m, 4H), 3.17-3.02 (m, 2H), 3.08 (s, 3H), 2.61 (dd, *J* = 12.8 Hz, 7.4 Hz, 1H), 2.46-2.43 (m, 1H), 2.30 (dd, *J* = 12.9 Hz, 10.1 Hz, 1H), 2.18-2.08 (m, 2H), 1.75-1.65 (m, 2H), 1.11 (s, 9H), 0.98 (s, 9H).

**Example 4: Preparation of Crystal Form A of Compound of Formula (I)**

Method 1:

270 g of the 2-methyltetrahydrofuran solvate of the compound of formula (I) was added to a mixed solvent of 540 mL of isopropyl acetate and 5.4 L of *n*-heptane. The mixture was heated to 55-65 °C, slurried for crystal transition for 12 h, slowly cooled to 20-30 °C, stirred for 1 h with the temperature maintained, filtered, and dried to give a solid (215 g). The obtained solid was confirmed to be the crystal form A of the compound of formula (I) by nuclear magnetic resonance and X-ray powder diffraction. The XRPD pattern of the crystal form A is shown in FIG. 5, the DSC pattern thereof is shown in FIG. 6, the TGA pattern is shown in FIG. 7, and the positions of XRPD diffraction peaks are shown in Table 3 below.

Table 3

| Peak No. | $2\theta$ value [° or degree] | d [Å] | Relative intensity (%) |
|---|---|---|---|
| 1 | 9.29 | 9.52 | 19 |
| 2 | 10.27 | 8.61 | 49 |
| 3 | 10.88 | 8.13 | 96 |
| 4 | 10.97 | 8.06 | 77 |
| 5 | 11.86 | 7.46 | 71 |
| 6 | 14.27 | 6.20 | 27 |
| 7 | 14.92 | 5.93 | 47 |

(continued)

| Peak No. | 2θ value [° or degree] | d [Å] | Relative intensity (%) |
|---|---|---|---|
| 8 | 15.09 | 5.87 | 79 |
| 9 | 15.61 | 5.67 | 33 |
| 10 | 15.78 | 5.61 | 30 |
| 11 | 16.61 | 5.33 | 73 |
| 12 | 17.67 | 5.02 | 81 |
| 13 | 18.28 | 4.85 | 94 |
| 14 | 18.50 | 4.79 | 75 |
| 15 | 18.85 | 4.70 | 13 |
| 16 | 19.16 | 4.63 | 15 |
| 17 | 20.09 | 4.42 | 72 |
| 18 | 20.64 | 4.30 | 100 |
| 19 | 21.48 | 4.13 | 19 |
| 20 | 22.03 | 4.03 | 25 |
| 21 | 22.18 | 4.01 | 36 |
| 22 | 22.44 | 3.96 | 17 |
| 23 | 22.97 | 3.87 | 16 |
| 24 | 23.80 | 3.74 | 34 |
| 25 | 25.63 | 3.47 | 31 |
| 26 | 29.59 | 3.02 | 14 |
| 27 | 30.26 | 2.95 | 14 |
| 28 | 31.30 | 2.86 | 12 |
| 29 | 34.46 | 2.60 | 16 |
| 30 | 34.50 | 2.60 | 15 |
| 31 | 36.69 | 2.45 | 8 |
| 32 | 38.06 | 2.36 | 8 |

$^1$H NMR (400 MHz, DMSO-$d_6$): δ 9.47 (d, *J* = 8.4 Hz, 1H), 9.06 (d, *J* = 8.6 Hz, 1H), 7.68 (s, 1H), 5.00-4.94 (m, 1H), 4.53 (d, *J* = 8.5 Hz, 1H), 4.33 (dd, *J* = 9.8 Hz, 7.1 Hz, 1H), 4.19 (d, *J* = 10.8 Hz, 1H), 3.92 (d, *J* = 11.0 Hz, 1H), 3.44-3.32 (m, 4H), 3.17-3.02 (m, 2H), 2.61 (dd, *J* = 12.7 Hz, 5.6 Hz, 1H), 2.50-2.43 (m, 1H), 2.30 (dd, *J* = 12.8 Hz, 10.0 Hz, 1H), 2.19-2.08 (m, 2H), 1.75-1.68 (m, 2H), 0.98 (s, 9H).

Method 2:

10 mg of the methyl *tert*-butyl ether solvate of the compound of formula (I) was dissolved in 50 μL of ethyl acetate, and 50 μL of n-heptane was added. The solution became turbid, and the resulting solid after the solvent was volatilized was identified by X-ray powder diffraction as crystal form A of the compound of formula (I).

Method 3:

15 mg of the methyl *tert*-butyl ether solvate of the compound of formula (I) was dissolved in 150 μL of isopropanol, and 400 μL of *n*-heptane was added. The solution became turbid, and was stirred for another 2 h. 40 μL of *n*-heptane was supplemented. The solution became turbid, and the resulting solid after the solvent was volatilized was identified by X-ray powder diffraction as crystal form A of the compound of formula (I).

**Test Examples for Biological Activity and Related Properties**

Test Example 1-1: Test of Inhibitory Activity against SARS-CoV-2 3CLpro by Compound of Formula (I)

The inhibitory activity of the compound of formula (I) against enzymatic activity of SARS-CoV-2 $3CL^{pro}$ was evaluated by fluorescence resonance energy transfer. The volume of the entire enzymatic reaction system was 120 μL, the final concentration of the protease was 30 nM, and the final concentration of the substrate was 20 μM. The buffer of the reaction system included 50 mM Tris pH 7.3 and 1 mM EDTA. SARS-CoV-2 $3CL^{pro}$ protease and compounds at different concentrations were added into a 96-well plate, and the plate was incubated at 30 °C for 10 min. The substrate was then added, and the plate was promptly placed in a microplate reader for reading. The excitation light and the emission light were 320 nM and 405 nM, respectively. The test time was 3.5 min, and fluorescence values were read every 35 s. The reaction rates were obtained by fitting the readings of the first 2 min of the final results and were compared with that of the control group (DMSO), and the inhibition rates were calculated. The $IC_{50}$ values and inhibition rate curves were obtained by fitting using GraphPad Prism 8 software.

The experimental results showed that the compound of formula (I) exhibited a potent inhibitory effect on SARS-CoV-2 $3CL^{pro}$, with an $IC_{50}$ value of < 0.1 μM.

**Test Example 1-2: Test of Inhibitory Activity against Mutant 3CL Protease in SARS-CoV-2 Omicron Strain by Compound of Formula (I)**

Experimental principle: The inhibitory effect of the compound of formula (I) disclosed herein on the activity of the mutant 3CL protease (P132H) in the Omicron strain was investigated using a method in which an enzyme reacts with a substrate to produce fluorescence resonance energy transfer (FRET).

The experimental materials are shown in the table below:

| Reagent material | Brand | Catalog No. |
|---|---|---|
| 3CL Protease-P132H mutant | Shanghai Panchao | / |
| Dabcyl-KTSAVLQSGFRKME-Edans (Coronavirus main protease fluorescent substrate) | Beyotime | P9733 |
| DTT (dithiothreitol) | Invitrogen | P2325 |
| BSA (bovine serum albumin) | Sigma | V900933 |
| EDTA (ethylenediaminetetraacetic acid) | Invitrogen | AM9260G |
| Tris-HCl (tris(hydroxymethyl)aminomethane) | Sangon Biotech | B548127 |

Experimental instruments and equipment:

| Instrument | Brand | Model |
|---|---|---|
| Echo nano-liter scale acoustic liquid handling system | Labcyte | Echo 650 |
| Flexstation 3 microplate reader | MolecMar Devices | FLEX3 |
| Centrifuge | Eppendorf | 5810 |

Experimental procedures:

A reaction buffer was prepared, which contained 20 mM Tris-HCl, 1 mM EDTA, 0.01% BSA, 1 mM DTT, and 100 mM NaCl. The test compounds were diluted to different concentrations in dimethyl sulfoxide (DMSO) using the Echo liquid handling system and transferred to a 384-well plate. The mutant 3CL protease was diluted with a reaction buffer and added to the 384-well plate at 10 μL/well. The plate was centrifuged at 1000 rpm for 1 min and then incubated at room temperature for 30 min. A substrate was then added at 10 μL/well, and the plate was centrifuged at 1000 rpm for 30 s to start the enzymatic reaction. In the reaction system, the final concentration of the enzyme was 50 nM, the final concentration of the substrate was 20 μM, and the concentration range of the compound was 10000 nM to 0.51 nM. Then the Kinetic Reduction Vmax mode was selected on the Flexstation 3 microplate reader, and the fluorescence values at the wavelength of 490 nm were continuously read every 75 s for 35 times to obtain the reaction rate values (V). The inhibition rates were calculated, and the half maximal inhibitory concentrations ($IC_{50}$) were obtained by four-parameter fitting using XLfit software. The calculation method of inhibition rate is as follows:

$$\text{Inhibition rate} = (V_{max} - V_{compound})/(V_{max} - V_{min}) \times 100\%$$

$V_{max}$ is the reaction rate value of wells containing only enzyme and substrate, $V_{min}$ is the reaction rate value of wells containing only substrate, and $V_{Compound}$ is the reaction rate value of wells containing test compound, enzyme and substrate.

Experimental results: The compound of formula (I) retained significant inhibitory activity against the 3CL protease having a P132H mutation in SARS-CoV-2 Omicron strain.

**Table 4. Inhibitory effect of compound of formula (I) on activity of 3CL protease in SARS-CoV-2 Omicron strain**

| 3CL protease | Compound of formula (I) $IC_{50}$ (μM) (n = 3*) |
|---|---|
| SARS-CoV-2 (Omicron strain) | 0.022±0.00090 |
| * Refers to three independent replicates. | |

**Test Example 2: Test of Inhibitory Activity against Coronavirus 3CL Protease of Different Sources by Compound of Formula (I)**

Experimental objective: to study the inhibitory effects of the compound of formula (I) on the activity of 3CL protease derived from six other coronaviruses capable of infecting humans. The six viruses are SARS-CoV, MERS-CoV, H229E-CoV, HKU1-CoV, NL63-CoV, and OC43-CoV.

Materials:

3CL protease: A recombinant full-length coronavirus 3CL protease was prepared in-house according to the genome sequences of coronaviruses, and the genome GenBank numbers of the SARS-CoV, MERS-CoV, H229E-CoV, HKU1-CoV, NL63-CoV, and OC43-CoV used were AAP13442.1, MT387202.1, AF304460.1, AY597011.2, AY567487.2, and AY903459.1, respectively. The DNA sequences required for protein expression of the six coronavirus 3CL proteases were purchased from Nanjing GenScript Biotech Co., Ltd.

The 3CL protease substrate was purchased from Nanjing GenScript Biotech Co., Ltd.

The chymotrypsin substrate was purchased from GL Biochem.

Other reagents are shown in the table below:

| Reagent material | Brand | Catalog No. |
|---|---|---|
| Bovine pancreas-derived chymotrypsin | Sigma | C4129 |
| Tris | Sigma | BCBX3837 |
| EDTA | Sigma | 0001434776 |

Experimental procedures:

A reaction buffer (containing 50 mM Tris and 1 mM EDTA) was prepared. The test compound was dissolved in DMSO to obtain a 100 mM stock solution, which was further diluted in a 2-fold gradient with the reaction buffer to obtain a total of 11 concentrations. 3CL protease and compounds at different concentrations were added into a 96-well plate, and the plate was incubated at room temperature for 10 min. The substrate was then added, and the plate was promptly placed in a microplate reader for reading. The volume of the entire enzymatic reaction system was 120 μL, the final concentrations of the SARS-CoV, MERS-CoV, H229E-CoV, HKU1-CoV, NL63-CoV and OC43-CoV proteases were 30 nM, 80 nM, 30 nM, 20 nM, 30 nM and 10 nM, respectively, and the final concentration of the substrate was 10 μM. During the reading, the wavelengths of the excitation light and the emission light were 340 nm and 490 nm, respectively. The test duration was 10 min, with fluorescence readings taken every 1 min. The final results were taken from the readings obtained within the first 5 min for fitting to obtain the reaction rates. The inhibition rate was then calculated using the following formula: inhibition rate = 1 - (reaction rate of test group/reaction rate of control group).

Experimental results: As shown in Table 5, the compound of formula (I) exhibited a relatively good inhibitory effect on 3CL proteases derived from six other coronaviruses, suggesting that the compound of formula (I) may have a broad-spectrum anti-coronavirus activity.

**Table 5. Inhibitory effect of the compound of formula (I) on 3CL proteases derived from other coronaviruses**

| 3CL protease source | Compound of formula (I) $IC_{50}$ ($\mu$M) |
|---|---|
| HKU1-CoV | 0.0049 |
| OC43-CoV | 0.010 |
| SARS-CoV | 0.024 |
| MERS-CoV | 0.060 |
| H229E-CoV | 0.13 |
| NL63-CoV | 0.85 |

**Test Example 3: Inhibitory Effect of Compound of Formula (I) on SARS-CoV-2 Vero E6 Prototype** Strain **(WIV04), Delta Strain (B.1.617.2), and Omicron Strain (B.1.1.529) at Cell Level**

Experimental objective: In this experiment, the inhibitory effect of the compound of formula (I) on the replication of SARS-CoV-2 prototype strain (strain WIV04), Delta strain (B.1.617.2), and Omicron strain (B.1.1.529) in Vero E6 cells was investigated by real-time fluorescence quantitative PCR detection of the viral copy number in the culture supernatant. Since Vero E6 cells highly express the efflux transporter P-gp, 0.5 $\mu$M of the P-gp inhibitor CP-100356 was added for co-incubation with the compound.

Materials:

Vero E6 cells were purchased from ATCC (Cat. No. CRL-1586). The SARS-CoV-2 prototype strain (SARS-CoV-2-WIV04 strain), Delta strain (B.1.617.2), and Omicron strain (B.1.1.529) viruses were sourced from the Microorganisms and Viruses Culture Collection Center, Wuhan Institute of Virology, Chinese Academy of Sciences.

Other reagents are shown in the table below:

| Reagent name | Brand | Catalog No. |
|---|---|---|
| TaKaRa MiniBEST Viral RNA/DNA Extraction Kit Ver.5.0 | Takara | 9766 |
| TaKaRa PrimeScript™ RT reagent Kit with gDNA Eraser | Takara | RR047A |
| TaKaRa SYBR® Premix Ex Taq ™ II | Takara | RR820A |
| Fetal bovine serum | Gibco | R2768 |
| DMEM medium | Gibco | C11995500BT |
| CCK8 | Beyotime | C0039 |
| Chloroquine phosphate | SIGMA | C6628-50G |
| Trypsin | BIOSHARP | BL512A |

Experimental instrument:

Biosafety cabinet (AC2-3S1, ESCO, Singapore)
Carbon dioxide incubator (Thermo Scientific HERAcell 150i, Thermo Scientific, USA)
Water purifier (Shenyuan SYS ultra-pure water system, Chengdu)
StepOne Plus Real-time PCR system (4376600, ABI, USA)
TC20™ Automated Cell Counter (1450102, BIO-RAD, USA)
T100™ Thermal Cycler (1861096, BIO-RAD, USA)
Centrifuge (Micro21/21R Thermo Scientific, USA)

Experimental procedures:

Vero E6 cells were digested with trypsin, placed in a medium (90% DMEM, 10% fetal bovine serum), and seeded

into a 48-well plate at 50000 cells/well, and the plate was incubated overnight. The test compound was dissolved in DMSO to prepare a 40 mM stock solution, which was further diluted in a gradient with a medium containing 0.5 μM of a Pgp inhibitor to obtain the concentration required for the test. The final concentration range of the test compound in the experiment was 1 μM to 0.004 μM. The cell supernatant was removed, the diluted compound (containing 0.5 μM of Pgp inhibitor) was added to each well, and the mixture was incubated for 1 h. Different strains of SARS-CoV-2 with a multiplicity of infection (MOI) of 0.01 or 0.001 were added to a biosafety level 3 (BSL-3) laboratory, and the mixture was incubated for 1 h. The supernatant was removed, and the plate was washed with PBS. The diluted compound (containing 0.5 μM of Pgp inhibitor) was added at 200 μL/well, and the supernatant was collected 24 h or 72 h after infection. The virus RNA in the supernatant was extracted and the virus copy number in the supernatant was detected using a real-time fluorescent quantitative PCR method. The inhibition rate of the compound was calculated based on the virus copy number, and the $IC_{50}$ of the compound was calculated using GraphPad Prism 8.

In the cytotoxicity assay, Vero E6 cells were digested, placed in a medium (90% DMEM, 10% fetal bovine serum), and seeded into a 96-well plate at 20000 cells/well, and the plate was incubated overnight. The test compound was dissolved in DMSO to prepare a 40 mM stock solution, which was further diluted in a gradient with a medium or a medium containing 0.5 μM of a Pgp inhibitor to obtain the concentration required for the test. The final concentration range of the test compound in the experiment was 500 μM to 1.95 μM. The cell supernatant was removed from the 96-well plate, and a medium containing the test compound (single-drug or containing 0.5 μM of the Pgp inhibitor) was added at 100 μL/well. After 24 h of incubation, the cell viability was measured using a CCK8 assay kit, and the inhibition rate and 50% cytotoxic concentration ($CC_{50}$) were calculated.

Experimental results: As shown in Table 6, the compound of formula (I), when combined with the P-gp inhibitor CP-100356, could dose-dependently inhibit the replication of the Delta strain in Vero E6 cells, with an $IC_{50}$ value of 0.040 μM. The compound of formula (I) in combination with the P-gp inhibitor also exhibited a strong inhibitory effect in the prototype strain, with an $IC_{50}$ of 0.027 μM. In addition, the compound of formula (I) in combination with the P-gp inhibitor could significantly inhibit the replication of the Omicron strain in Vero E6 cells with an $IC_{50}$ of 0.12 μM. The compound of formula (I), single drug or in combination with the P-gp inhibitor, had no significant cytotoxicity on Vero E6 cell proliferation, with $CC_{50}$ > 500 μM.

**Table 6. Inhibitory effect of compound of formula (I) in combination with P-gp inhibitor on SARS-CoV-2 in Vero E6 cells**

| Experiment | Compound of formula (I) + 0.5 μM CP-100356 | |
|---|---|---|
| | $IC_{50}$ (μM) | Selection index SI SI=($CC_{50}$/$IC_{50}$) |
| Vero E6 prototype strain (WIV04) | 0.027 (mean, n = 2) | >18519 |
| Vero E6 Delta strain (B.1.617.2) | 0.040 (mean, n = 2) | >12500 |
| Vero E6 Omicron strain (B.1.1.529) | 0.12 | >4167 |
| Vero E6 single-drug $CC_{50}$ | >500 μM | |
| Vero E6 in combination with P-gp inhibitor $CC_{50}$ | >500 μM | |

**Test Example 4: *In Vivo* Antiviral Effect of Compound of Formula (I) on SARS-CoV-2 Delta Strain in hACE2-K18 Transgenic Mice**

Experimental objective: This study evaluated the antiviral activity of the compound of formula (I) against the SARS-CoV-2 Delta strain in K18 transgenic mice stably expressing human angiotensin-converting enzyme 2 (ACE2) (K18-hACE2).

Materials:

K18-hACE2 transgenic mice aged 7-8 weeks were purchased from Jiangsu GemPharmatech Co., Ltd. SARS-CoV-2 Delta strain virus was sourced from the Microorganisms and Viruses Culture Collection Center, Wuhan Institute of Virology, Chinese Academy of Sciences.

Ritonavir was purchased from Shanghai Desano Chemical Pharmaceutical Co., Ltd.
Vero E6 cells were purchased from ATCC (Cat. No. CRL-1586).

Other reagents are shown in the table below:

| Reagent name | Brand | Catalog No. |
|---|---|---|
| Qiagen 74106 RNeasy Mini Kit | Qiagen | 74106 |
| TaKaRa PrimeScript™ RT reagent Kit with gDNA Eraser | Takara | RR047A |
| TaKaRa SYBR® Premix Ex Taq ™ II | Takara | RR820A |
| Fetal Bovine Serum | Gibco | 10099-141C |
| DMEM medium | Gibco | C11995500BT |
| Tissue fixative solution | BOSTER | AR1068 |
| Aquacide II (sodium methylcellulose) | Millipore | 17851 |
| DMEM medium (powder, high sugar) | Gibco | 12100046 |
| Crystal violet | Sinopharm | 71012314 |

Experimental instrument:

Biosafety cabinet (AC2-3S1, ESCO, Singapore)
Carbon dioxide incubator (Thermo Scientific HERAcell 150i, Thermo Scientific, USA)
Water purifier (Shenyuan SYS ultra-pure water system, Chengdu)
StepOne Plus Real-time PCR system (4376600, ABI, USA)
TC20™ Automated Cell Counter (1450102, BIO-RAD, USA)
T100™ Thermal Cycler (1861096, BIO-RAD, USA)
Centrifuge (Micro21/21R Thermo Scientific, USA)
Tissue grinding instrument (JXFSTPRP-CL, Shanghai Jingxin, China)

Experimental procedures: K18-hACE2 transgenic mice were infected with SARS-CoV-2 Delta strain by nasal drop on day 0. 2 h after infection, the vehicle and 50 mg/kg or 200 mg/kg of the compound of formula (I) (in combination with 50 mg/kg of the cytochrome P450 inhibitor ritonavir) were intragastrically administered BID for 2 days (wherein the administration was performed once on day 0, twice on day 1, and once on day 2) or 4 days (wherein the administration was performed once on day 0 and twice on days 1, 2, and 3). Changes in the body weight of the mice were recorded, and lung and brain tissues were collected at the endpoint. The left lung was fixed with formaldehyde and then subjected to embedding, sectioning and H&E staining for histopathological examination. The right lung and brain tissues were divided into two parts. One part was ground, and the homogenate was taken to extract RNA and carry out reverse transcription. The copy number of the virus was detected by real-time fluorescence quantitative PCR. The other part was ground, and the homogenate was taken for detecting the virus titer by a plaque assay. Plaque assay method: Vero E6 cells were seeded in a 24-well plate at 12000 cells per well and cultured overnight. The tissue homogenate stock solution was serially diluted 10-fold in DMEM medium for later use. The cell supernatant was removed, and the diluted tissue homogenate was added. The mixture was incubated for 1 h, and then the supernatant was removed. A medium containing 1% sodium methylcellulose and 2% FBS was added, and the mixture was cultured for 4 days. The medium was then removed, and after fixation with paraformaldehyde, the cells were stained with 1% (w/v) crystal violet, and the number of plaques in each well was counted.

Test results: As shown in Table 7, 2 days after infection, the compound of formula (I) at 50 mg/kg and 200 mg/kg in combination with ritonavir significantly reduced the viral load in the lungs compared with the model group (the average viral copy number was $9.19 \pm 0.30$ log10 copies/g), and the average copy number was separately $7.66 \pm 0.27$ log10 copies/g and $6.79 \pm 0.30$ log10 copies/g, wherein the viral copy number was reduced by 2.4 log10 copies/g at the dose of 200 mg/kg. A persistent inhibition of viral copy number by the compound of formula (I) was observed after 4 days of infection.

In terms of the viral titer, as shown in FIG. 8, a significant inhibitory effect was observed for the compound of formula (I). 2 days after infection, the virus replication was completely inhibited at the dose of 200 mg/kg, and the titer was not detected. Compared to the model group, the viral titer was reduced by more than 3 log10 PFU/g at 50 mg/kg, and 4 days after infection, the compound of formula (I) showed a sustained inhibitory effect on the viral titer. The body weight is shown in FIG. 9. 4 days after infection, the body weight of the mice in the model group was reduced by about 10%, while the body weight of the administration group of the compound of formula (I) was not significantly reduced, indicating that the continuous administration of the compound of formula (I) showed no significant toxicity. We further detected the viral loads in the brains of the mice, and found no significant infections in the groups 2 days after infection. On day 4 after infection, the viral copy number in the brains of the mice was significantly reduced by the compound of formula (I) at both 50 mg/kg and

200 mg/kg as compared to the model group, and particularly, the viral copy number in the brains of the mice at the dose of 200 mg/kg was comparable to that of the uninfected normal group. We further detected the virus titer in the brains 4 days after infection, and the results are shown in FIG. 10. Compared with the model group, no virus titer was detected for the compound of formula (I) at both doses, indicating a strong inhibitory effect of the compound of formula (I). In addition, histopathological analysis of the lungs showed that compared with the model group, the compound of formula (I) at the dose of 200 mg/kg significantly ameliorated lung damage, including reducing the degree of alveolar atrophy or expansion and the degree of alveolar membrane thickening.

**Table 7. Viral loads in lungs and brains of mice 2 days and 4 days after infection (mean ± SD)**

| Group | Lung tissue (log10 copies/g) | | Brain tissue (log10 copies/mg) | |
|---|---|---|---|---|
| | Day 2 | Day 4 | Day 2 | Day 4 |
| Model group | 9.19 ± 0.30 | 9.35 ± 0.30 | 1.34±0.78 | 7.17 ± 0.27 |
| Compound of formula (I) - 200 mg/kg + ritonavir - 50 mg/kg | 6.79 ± 0.30 | 7.48 ± 0.63 | 1.04±0.24 | 0.99 ± 0.42 |
| Compound of formula (I) - 50 mg/kg + ritonavir - 50 mg/kg | 7.66 ± 0.27 | 7.88 ± 0.74 | 0.77±0.20 | 2.88 ± 1.26 |
| Normal group | 5.76 ± 0.32 | 6.27 ± 0.18 | 1.08±0.06 | 1.08 ± 0.06 |

**Test Example 5: Selectivity of Compound of Formula (I) against Kinases**

Experimental objective: The inhibitory activity of the compound of formula (I) against 413 kinases was determined on the KinaseProfile experimental platform to study the selectivity of the compound of formula (I) against kinases.

Materials:

Full Human Panel [10 μM ATP] KinaseProfiler is a test product provided by Eurofins, Catalog No. 50-005KP10. This product contains 413 kinases.

Experimental procedures:

Each of the selected kinases was subjected to compound testing using Eurofins standard KinaseProfiler analytical method and following the relevant standard operating procedure. Protein kinases were detected by radiation method, while lipid kinases were detected by HTRF method. The concentration of ATP in the experiment was 10 μM. Details of each kinase are available on the Eurofins website at the following address: https://www.eurofinsdiscoveryservices.com/catalogmanagement/viewItem/Full-Human-Panel-10-uM-ATP-KinaseProfiler/50-005KP10.

Experimental results: For 413 kinases, the inhibition rates of the compound of formula (I) were all less than 30% at the concentration of 10 μM, and no significant inhibitory effect was observed, suggesting that the compound of formula (I) has excellent selectivity.

**Test Example 6: Selectivity of Compound of Formula (I) against Safety Targets**

Experimental objective: The effect of the compound of formula (I) on 47 safety-related targets was detected on the Safetyscan experimental platform.

Materials:

Safety47 Panel Dose Response SAFETYscan is a test product provided by Eurofins, Cat. No. 87-1003DR. This product contains 78 tests related to 47 safety targets.

Experimental procedures:

For 78 tests related to 47 safe targets, the experimental methods used included: cAMP assay, calcium flux assay,

hormone nuclear receptor assay, kinase binding assay, enzyme activity assay, neurotransmitter transporter assay, ion channel assay, and transporter assay. Specific methods for each experiment are available at the website of eurofins, which can be found at https://www.eurofinsdiscoveryservices.com/catalogmanagement/viewItem/Safety47-Panel-Dose-Response-SAFETYscan-DiscoverX/87-1003DR.

Experimental results: for 47 safety-related targets, the compound of formula (I) had no significant inhibition or activation effect ($EC_{50}$ was all greater than 100 μM) at the concentration of 100 μM, suggesting that the compound of formula (I) has excellent selectivity.

**Test Example 7: Human Plasma Protein Binding Assay of Compound of Formula (I)**

**Materials**

Human plasma was purchased from BioIVT, anticoagulated with EDTA K2, and stored at -80 °C. The 96-well equilibrium dialysis plate was purchased from HTDialysis LLC. The equilibrium dialysis membrane was purchased from Gales Ferry.

**Experimental procedures**

An alkaline solution with a concentration of 14.2 g/L disodium hydrogen phosphate and 8.77 g/L sodium chloride was prepared with ultrapure water, and the alkaline solution could be stored at 4 °C for 7 days. An acidic solution with a concentration of 12.0 g/L sodium dihydrogen phosphate and 8.77 g/L sodium chloride was prepared with ultrapure water, and the acidic solution could be stored at 4 °C for 7 days. The alkaline solution was titrated with the acidic solution until the pH value was 7.4, and the buffer could be stored at 4 °C for 7 days. On the day of the experiment, the pH value of the buffer was measured, and if the pH value exceeded the range of 7.4+0.1, the pH value was adjusted.

The dialysis membrane was soaked in ultrapure water for 60 min to separate the membrane into two pieces, then soaked with 20% ethanol for 20 min, and finally soaked with dialysis buffer for 20 min.

The frozen plasma was quickly thawed at room temperature.

The plasma was then centrifuged at 4 °C for 10 min at a centrifugal force of 3,220 g to remove clots, and the supernatant was collected into a new centrifuge tube. The pH of the plasma was measured and recorded.

A 10 mM DMSO stock solution of the test substance was prepared. 2 μL of the stock solution (10 mM) was diluted with 98 μL of DMSO to give a working solution (200 μM). 3 μL of the working solution was taken, and 597 μL of human plasma was added to make a final concentration of 1 μM (0.5% DMSO). The mixture was vortexed to be mixed well.

120 μL of the plasma sample containing the compound was added to one side of the dialysis membrane, and a dialysate (phosphate-buffered saline) in the same volume was added to the other side. The experiment was conducted in duplicate. The dialysis plate was sealed, placed in an incubation device, and incubated at approximately 100 rpm at 37 °C with 5% $CO_2$ for 6 h. After the incubation was completed, the film was removed, and 50 μL was pipetted from the buffer side and the plasma side of each well to different wells of a new plate.

50 μL of blank plasma was added to the phosphate-buffered saline sample. A blank phosphate-buffered saline in the same volume was added to the plasma sample. 300 μL of room-temperature quencher (containing internal standard acetonitrile (IS, 500 nM labetalol, 100 nM alprazolam, and 2 μM ketoprofen)) was added to precipitate the protein. The mixture was vortexed for 5 min. The mixture was centrifuged at 3220 g at 4 °C for 30 min. 100 μL of the supernatant was transferred to a new plate. The supernatant was diluted with 100 μL or 200 μL of water based on the liquid chromatography-mass spectrometry response signal and the peak shape of the test substance. The mixture was well mixed, and the sample was analyzed using liquid chromatography-mass spectrometry.

All calculations were performed by Microsoft Excel. The peak areas of the test substance on the buffer side and the plasma side were determined. The formula for calculating the plasma protein binding rate of the test compound and the control drug is as follows: free rate = (ratio of sample peak area to internal standard peak area on buffer side/ratio of sample peak area to internal standard peak area on plasma side) × 100%, binding rate = 1 - free rate, recovery rate = (ratio of sample peak area to internal standard peak area on buffer side + ratio of sample peak area to internal standard peak area on plasma side)/(ratio of sample peak area to internal standard peak area in initial plasma sample) × 100%. The ratio of sample peak area to internal standard peak area on buffer side represents the free concentration of the compound, the ratio of sample peak area to internal standard peak area on plasma side represents the sum of the free concentration and binding concentration of the compound, and the ratio of sample peak area to internal standard peak area in initial plasma sample represents the total concentration of the compound at the initiation of sample incubation.

**Results:**

See Table 8. After incubation at 37 °C for 6 h, the compound of formula (I) at 1 μM showed an average free rate of

46.63%, a binding rate of 53.37%, and a recovery rate of 88.02%.

**Table 8. Results of human plasma protein binding assay of compound of formula (I)**

| Compound | Free rate (F%) | | | Binding rate (%) | Recovery rate (%) |
|---|---|---|---|---|---|
| | Replicate 1 | Replicate 2 | Mean value | | |
| Compound of formula (I) | 48.71 | 44.55 | 46.63 | 53.37 | 88.02 |

**Test Example 8: Tissue Distribution Assay of Compound of Formula (I) Following Single Intragastric Administration**

Materials:

60 Balb/c mice (purchased from Shanghai Minchang Biotechnology Co., Ltd.) were provided, half male and half female, weighing 18-25 g.

Experimental procedures:

Balb/c mice were given the compound of formula (I) by a single intragastric administration at a dose of 100 mg/kg and a volume of 10 mL/kg.

Before administration and 5 min, 0.25 h, 1.0 h, 2.0 h, 3.0 h, 5.0 h, 7.0 h and 10 h after administration (6 mice at each time point, half male and half female); 0.2 mL of blood was collected from the retrobulbar venous plexus at the time points set above, placed into an EDTA-K2 test tube, and centrifuged at 11000 rpm for 5 min. The plasma was separated and frozen in a refrigerator at -70 °C; at the time points of 0.25 h, 1.0 h, 3.0 h and 7.0 h, the whole blood was collected and the mice were immediately dissected to collect lung tissues. The tissues were washed with cold physiological saline to remove the residual blood and contents on the surface, dried, labeled and stored at -70 °C for later testing. The content of the compound of formula (I) in plasma and lung tissue was determined by LC/MS-MS, and the lung/blood exposure ratio was calculated.

Results:

After a single intragastric administration of the compound of formula (I) to Balb/c mice, the ratio of lung tissue exposure to plasma exposure was 0.62, indicating that the compound of formula (I) had high exposure in lung tissues.

**Test Example 9: Safety Pharmacology Assay for Effect of Intragastric Administration of Compound of Formula (I) on Cardiovascular System in Cynomolgus Monkeys**

In the 2-week repeat-dose toxicity study in cynomolgus monkeys, the effect of the compound of formula (I) on the cardiovascular system was investigated concurrently.

Materials:

Thirty-two cynomolgus monkeys (half male and half female, aged 2.5-5 years) were used for administration.

Animal source: Yunnan Yingmao Biotechnology Co., Ltd.; Guangxi Xiongsen Primate Experimental Animal Breeding Development Co., Ltd.; Zhongke Lingrui (Zhanjiang) Biotechnology Co., Ltd.

Systolic blood pressure (SBP), diastolic blood pressure (DBP) and mean arterial pressure (MBP) of all conscious animals were measured using the intelligent non-invasive sphygmomanometer BP-98E using the Provantis/v10.2.3.1 electronic data capture system (PV-02).

Experimental procedures: Thirty-two cynomolgus monkeys were randomly grouped (group 1 and group 4: 5 animals/sex/group; group 2 and group 3: 3 animals/sex/group; 4 groups in total) and subjected to administration of the compound of formula (I) (40 mg/kg/day, 160 mg/kg/day, and 600 mg/kg/day) or the control formulation (98.9% vehicle formulation + 1.1% MTBE (methyl *tert-butyl* ether), 0 mg/kg/day) by nasogastric feeding twice daily for a total of 14 days, followed by a 14-day recovery period. All the animals were included in this study to evaluate the effects of the administration on ECG parameters (including heart rate, PR interval, QRS duration, QT interval and QTcF) and blood pressure in the pre-administration period, administration period and recovery period.

Experimental results: Under the conditions of this experiment, after the compound of formula (I) (40 mg/kg/day, 160 mg/kg/day, and 600 mg/kg/day) was intragastrically administered to the cynomolgus monkeys by nasogastric feeding

twice daily for 14 days, no test sample-related cardiovascular system changes were observed; no test sample-related arrhythmia was observed; no test sample-related changes in ECG parameters or blood pressure were observed throughout the entire experiment.

**Test Example 10: Chemical Stability Investigation Test**

Test method: The chemical stability of the crystal form A of the compound of formula (I) and the crystal form B of the methyl *tert-butyl* ether solvate of the compound of formula (I) was investigated under high temperature, illumination, acceleration, and high humidity conditions, and under illumination conditions, both unpackaged and packaged (packaged with two layers of polyethylene and one layer of pharmaceutical composite film) were investigated. The results are shown in Table 9. Test results: The crystal form B was left to stand under conditions of various influencing factors for 12 days, and the crystal form A was left to stand under conditions of various influencing factors for 12 days and 30 days. Both the crystal form A and the crystal form B were stable in chemical stability with no significant change in content.

**Table 9. Stability investigation test results**

| Compound | Storage condition | Store time | Initial purity | Purity |
|---|---|---|---|---|
| Crystal form B of methyl *tert*-butyl ether solvate of compound of formula (I) | Illumination, packaged | Day 12 | 98.41% | 98.52% |
| | Illumination, unpackaged | | | 98.49% |
| | 40°C, 75%RH | | | 98.48% |
| | 25°C, 92.5%RH | | | 98.51% |
| | 60°C | | | 98.53% |
| Crystal form A of compound of formula (I) | Illumination, packaged | Day 12 | 98.78% | 98.79% |
| | Illumination, unpackaged | | | 98.80% |
| | 40°C, 75%RH | | | 98.80% |
| | 25°C, 92.5%RH | | | 98.79% |
| | 60°C | | | 98.81% |
| | Illumination, packaged | Day 30 | | 98.75% |
| | Illumination, unpackaged | | | 98.69% |

**Test Example 11: Crystal Form Stability Investigation Test**

Test method:

The crystal form A of the compound of formula (I) was packaged and stored according to the following scheme, and the sample was taken out on day 30 for XRPD determination.

| Compound | Package | Storage condition | Store time |
|---|---|---|---|
| Crystal form A of compound of formula (I) | Transparent glass bottle, sealed | 40 °C/75% RH sealing | Day 30 |
| | Transparent glass bottle, sealed | 60°C | Day 30 |
| | Transparent glass bottle, sealed | Illumination of 4500 lx + ultraviolet (bare light) | Day 30 |
| | Transparent glass bottle, sealed, with an aluminum foil bag added outside | Illumination of 4500 lx + ultraviolet (in the dark) | Day 30 |
| | Transparent glass bottle, open | 92.5% RH high humidity | Day 30 |

The test results show that after the crystal form A of the compound of formula (I) was left to stand under conditions of various influencing factors for 30 days, the characteristic diffraction peaks of the crystal form were consistent, the crystallinity was not significantly different, and the crystal form was stable.

**Test Example 12: Hygroscopicity Test**

Test method:

(1) Experimental instrument: dynamic vapor sorption DVS Intrinsic PLUS.
(2) Experimental condition: The samples (proper amounts) were placed in DVS sample trays for testing.
(3) DVS parameters:
Temperature: 25 °C;
Balancing: dm/dt ≤ 0.002%/min
RH (%) test gradient: 10%
Range of RH (%) test gradient: 0%-90%-0%.
Hygroscopicity evaluation criteria (according to the description of hygroscopicity characteristics and the definitions of hygroscopic weight gains in "9103 Guidelines for Hygroscopicity" in Volume IV of the 2020 edition of Chinese Pharmacopoeia):

| Category of hygroscopicity | Hygroscopic weight gain* (ΔW%) |
|---|---|
| Deliquescence | Absorbing sufficient water to form a liquid |
| Very hygroscopic | 15% ≤ ΔW% |
| Hygroscopic | 2% ≤ ΔW% < 15% |
| Slightly hygroscopic | 0.2% ≤ ΔW% < 2% |
| Non-hygroscopic or hardly hygroscopic | ΔW% < 0.2% |
| *Hygroscopic weight gain at 25±1 °C and 80±2% RH. | |

Results:

The crystal form A of the compound of formula (I) had hygroscopic weight gains of only 0.5% and 0.6% under the conditions of 80% RH and 90% RH, respectively.

## Claims

1. A crystal of a compound of formula (I)

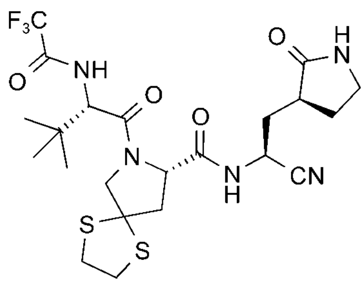

formula (I).

2. A crystal form A of a compound of formula (I),

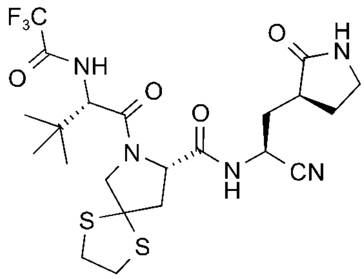

formula (I)

the X-ray powder diffraction pattern expressed in terms of diffraction angles 2$\theta$ of the crystal form A has diffraction peaks at 10.88±0.20°, 15.09±0.20°, 17.67±0.20°, 18.28±0.20°, and 20.64±0.20°; or

the X-ray powder diffraction pattern expressed in terms of diffraction angles 2$\theta$ of the crystal form A has diffraction peaks at 10.88±0.20°, 15.09±0.20°, 16.61±0.20°, 17.67±0.20°, 18.28±0.20°, 18.50±0.20°, 20.09±0.20°, and 20.64±0.20°; or

the X-ray powder diffraction pattern expressed in terms of diffraction angles 2$\theta$ of the crystal form A has diffraction peaks at 10.27±0.20°, 10.88±0.20°, 11.86±0.20°, 15.09±0.20°, 16.61±0.20°, 17.67±0.20°, 18.28±0.20°, 18.50±0.20°, 20.09±0.20°, and 20.64±0.20°; or

the X-ray powder diffraction pattern expressed in terms of diffraction angles 2$\theta$ of the crystal form A has diffraction peaks at 9.29±0.20°, 10.27±0.20°, 10.88±0.20°, 10.97±0.20°, 11.86±0.20°, 14.27±0.20°, 14.92±0.20°, 15.09±0.20°, 15.61±0.20°, 15.78±0.20°, 16.61±0.20°, 17.67±0.20°, 18.28±0.20°, 18.50±0.20°, 20.09 ±0.20°, 20.64±0.20°, 22.18±0.20°, 23.80±0.20°, and 25.63±0.20°; or

the X-ray powder diffraction pattern expressed in terms of diffraction angles 2$\theta$ of the crystal form A has diffraction peaks as shown in Table 3;

Table 3

| Peak No. | 2$\theta$ value [° or degree] | d [Å] | Relative intensity (%) |
|---|---|---|---|
| 1 | 9.29 | 9.52 | 19 |
| 2 | 10.27 | 8.61 | 49 |
| 3 | 10.88 | 8.13 | 96 |
| 4 | 10.97 | 8.06 | 77 |
| 5 | 11.86 | 7.46 | 71 |
| 6 | 14.27 | 6.20 | 27 |
| 7 | 14.92 | 5.93 | 47 |
| 8 | 15.09 | 5.87 | 79 |
| 9 | 15.61 | 5.67 | 33 |
| 10 | 15.78 | 5.61 | 30 |
| 11 | 16.61 | 5.33 | 73 |
| 12 | 17.67 | 5.02 | 81 |
| 13 | 18.28 | 4.85 | 94 |
| 14 | 18.50 | 4.79 | 75 |
| 15 | 18.85 | 4.70 | 13 |
| 16 | 19.16 | 4.63 | 15 |
| 17 | 20.09 | 4.42 | 72 |
| 18 | 20.64 | 4.30 | 100 |
| 19 | 21.48 | 4.13 | 19 |
| 20 | 22.03 | 4.03 | 25 |

(continued)

| Peak No. | 2θ value [° or degree] | d [Å] | Relative intensity (%) |
|---|---|---|---|
| 21 | 22.18 | 4.01 | 36 |
| 22 | 22.44 | 3.96 | 17 |
| 23 | 22.97 | 3.87 | 16 |
| 24 | 23.80 | 3.74 | 34 |
| 25 | 25.63 | 3.47 | 31 |
| 26 | 29.59 | 3.02 | 14 |
| 27 | 30.26 | 2.95 | 14 |
| 28 | 31.30 | 2.86 | 12 |
| 29 | 34.46 | 2.60 | 16 |
| 30 | 34.50 | 2.60 | 15 |
| 31 | 36.69 | 2.45 | 8 |
| 32 | 38.06 | 2.36 | 8 |

or
the crystal form A has the X-ray powder diffraction pattern expressed in terms of diffraction angles 2θ substantially as shown in FIG. 5.

**3.** The crystal form A of the compound of formula (I) according to claim 2, wherein the crystal form A has a DSC pattern with a peak at 213.78 °C ± 5.0 °C; or
the crystal form A has a DSC pattern substantially as shown in FIG. 6.

**4.** A method for preparing the crystal form A of the compound of formula (I) according to claim 2 or 3, comprising: mixing the compound of formula (I) with a solvent (i) and a solvent (ii), crystallizing, and separating out a solid, wherein the solvent (i) is selected from at least one of isopropyl acetate, ethyl acetate, and isopropanol, preferably isopropyl acetate, and the solvent (ii) is selected from at least one of n-hexane and n-heptane, preferably n-heptane.

**5.** A solvate of a compound of formula (I),

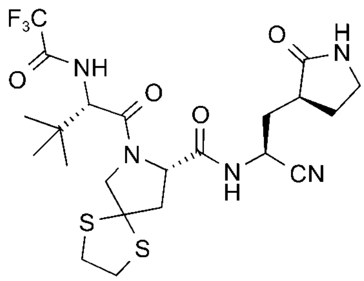

formula (I)

the solvate is selected from a methyl *tert-butyl* ether solvate and a 2-methyltetrahydrofuran solvate.

**6.** The solvate of the compound of formula (I) according to claim 5, wherein the molar ratio of the compound of formula (I) to the solvent is about 0.5-2, preferably about 0.8-1.2, and more preferably about 1.0.

**7.** A crystal form B of a methyl *tert-butyl* ether solvate of a compound of formula (I), wherein

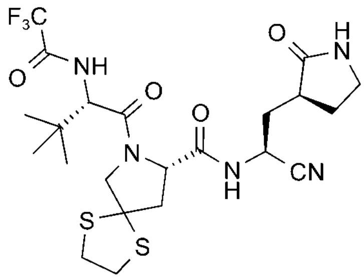

formula (I)

the X-ray powder diffraction pattern expressed in terms of diffraction angles 2$\theta$ of the crystal form B has diffraction peaks at 6.26±0.20°, 17.66±0.20°, and 20.24±0.20°; or
the X-ray powder diffraction pattern expressed in terms of diffraction angles 2$\theta$ of the crystal form B has diffraction peaks at 6.26±0.20°, 7.27±0.20°, 11.47±0.20°, 13.06±0.20°, 15.57±0.20°, 17.66±0.20°, 20.24±0.20°, and 22.72±0.20°; or
the X-ray powder diffraction pattern expressed in terms of diffraction angles 2$\theta$ of the crystal form B has diffraction peaks at 6.26±0.20°, 7.27±0.20°, 8.84±0.20°, 10.18±0.20°, 10.30±0.20°, 11.47±0.20°, 13.06±0.20°, 14.44 ±0.20°, 15.57±0.20°, 17.66±0.20°, 20.24±0.20°, 20.51±0.20°, and 22.72±0.20°; or
the X-ray powder diffraction pattern expressed in terms of diffraction angles 2$\theta$ of the crystal form B has diffraction peaks as shown in Table 2;

Table 2

| Peak No. | 2$\theta$ value [° or degree] | d [Å] | Relative intensity (%) |
|---|---|---|---|
| 1 | 6.26 | 14.11 | 44 |
| 2 | 7.27 | 12.15 | 15 |
| 3 | 8.84 | 10.00 | 5 |
| 4 | 10.18 | 8.68 | 5 |
| 5 | 10.30 | 8.58 | 5 |
| 6 | 11.47 | 7.71 | 11 |
| 7 | 12.00 | 7.37 | 7 |
| 8 | 12.42 | 7.12 | 8 |
| 9 | 13.06 | 6.78 | 14 |
| 10 | 14.44 | 6.13 | 12 |
| 11 | 15.43 | 5.74 | 14 |
| 12 | 15.57 | 5.69 | 19 |
| 13 | 17.66 | 5.02 | 100 |
| 14 | 17.95 | 4.94 | 19 |
| 15 | 20.24 | 4.38 | 41 |
| 16 | 20.33 | 4.36 | 30 |
| 17 | 20.51 | 4.33 | 23 |
| 18 | 22.72 | 3.91 | 23 |

or
the crystal form B has the X-ray powder diffraction pattern expressed in terms of diffraction angles 2$\theta$ substantially as shown in FIG. 3.

**8.** The crystal form B according to claim 7, wherein the crystal form B has a DSC pattern with peaks at 93.36±5.0 °C,

170.25±5.0 °C, and 214.18±5.0 °C; or
the crystal form B has a DSC pattern substantially as shown in FIG. 4.

**9.** A method for preparing the crystal form B according to claim 7 or 8, comprising: mixing the compound of formula (I) with methyl *tert*-butyl ether, and separating out a solid after stirring.

**10.** A crystal form C of a 2-methyltetrahydrofuran solvate of a compound of formula (I),

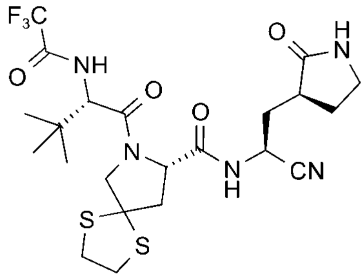

formula (I)

the X-ray powder diffraction pattern expressed in terms of diffraction angles 2*θ* of the crystal form C has diffraction peaks at 6.21±0.20°, 17.48±0.20°, and 20.86±0.20°; or
the X-ray powder diffraction pattern expressed in terms of diffraction angles 2*θ* of the crystal form C has diffraction peaks at 6.21±0.20°, 7.04±0.20°, 11.71±0.20°, 17.48±0.20°, 20.59±0.20°, and 20.86±0.20°; or
the X-ray powder diffraction pattern expressed in terms of diffraction angles 2*θ* of the crystal form C has diffraction peaks at 6.21±0.20°, 7.04±0.20°, 11.71±0.20°, 14.74±0.20°, 15.77±0.20°, 17.48±0.20°, 20.59±0.20°, and 20.86±0.20°; or
the X-ray powder diffraction pattern expressed in terms of diffraction angles 2*θ* of the crystal form C has diffraction peaks as shown in Table 1;

Table 1

| Peak No. | 2*θ* value [° or degree] | d [Å] | Relative intensity (%) |
|---|---|---|---|
| 1 | 6.21 | 14.22 | 25 |
| 2 | 7.04 | 12.55 | 13 |
| 3 | 8.70 | 10.15 | 6 |
| 4 | 11.71 | 7.55 | 16 |
| 5 | 12.42 | 7.12 | 8 |
| 6 | 14.13 | 6.27 | 5 |
| 7 | 14.74 | 6.01 | 15 |
| 8 | 14.98 | 5.91 | 6 |
| 9 | 15.77 | 5.62 | 18 |
| 10 | 17.23 | 5.14 | 12 |
| 11 | 17.48 | 5.07 | 100 |
| 12 | 17.93 | 4.94 | 7 |
| 13 | 20.06 | 4.42 | 8 |
| 14 | 20.59 | 4.31 | 23 |
| 15 | 20.86 | 4.25 | 34 |

or
the crystal form C has the X-ray powder diffraction pattern expressed in terms of diffraction angles 2θ substantially

as shown in FIG. 1.

**11.** The crystal form C according to claim 10, wherein the crystal form C has a DSC pattern with peaks at 90.39±5.0 °C, 167.59±5.0 °C, and 214.22±5.0 °C; or
the crystal form C has a DSC pattern substantially as shown in FIG. 2.

**12.** A method for preparing the crystal form C according to claim 10 or 11, comprising: mixing the compound of formula (I) with 2-methyltetrahydrofuran, stirring, and separating out a solid after crystallizing.

**13.** A pharmaceutical combination, comprising the crystal form A of the compound of formula (I) according to claim 2 or 3, the crystal form B according to claim 7 or 8, the crystal form C according to claim 10 or 11, or a combination thereof, and an additional antiviral drug, and preferably, the additional antiviral drug is ritonavir.

**14.** A pharmaceutical composition, comprising the crystal form A of the compound of formula (I) according to claim 2 or 3, the crystal form B according to claim 7 or 8, the crystal form C according to claim 10 or 11, or a combination thereof, and a pharmaceutically acceptable excipient.

**15.** Use of the crystal form A of the compound of formula (I) according to claim 2 or 3, the crystal form B according to claim 7 or 8, the crystal form C according to claim 10 or 11, or a combination thereof, or the pharmaceutical combination according to claim 13, or the pharmaceutical composition according to claim 14 in the preparation of a medicament for preventing or treating a related disease caused by infection with a coronavirus and/or a picornavirus.

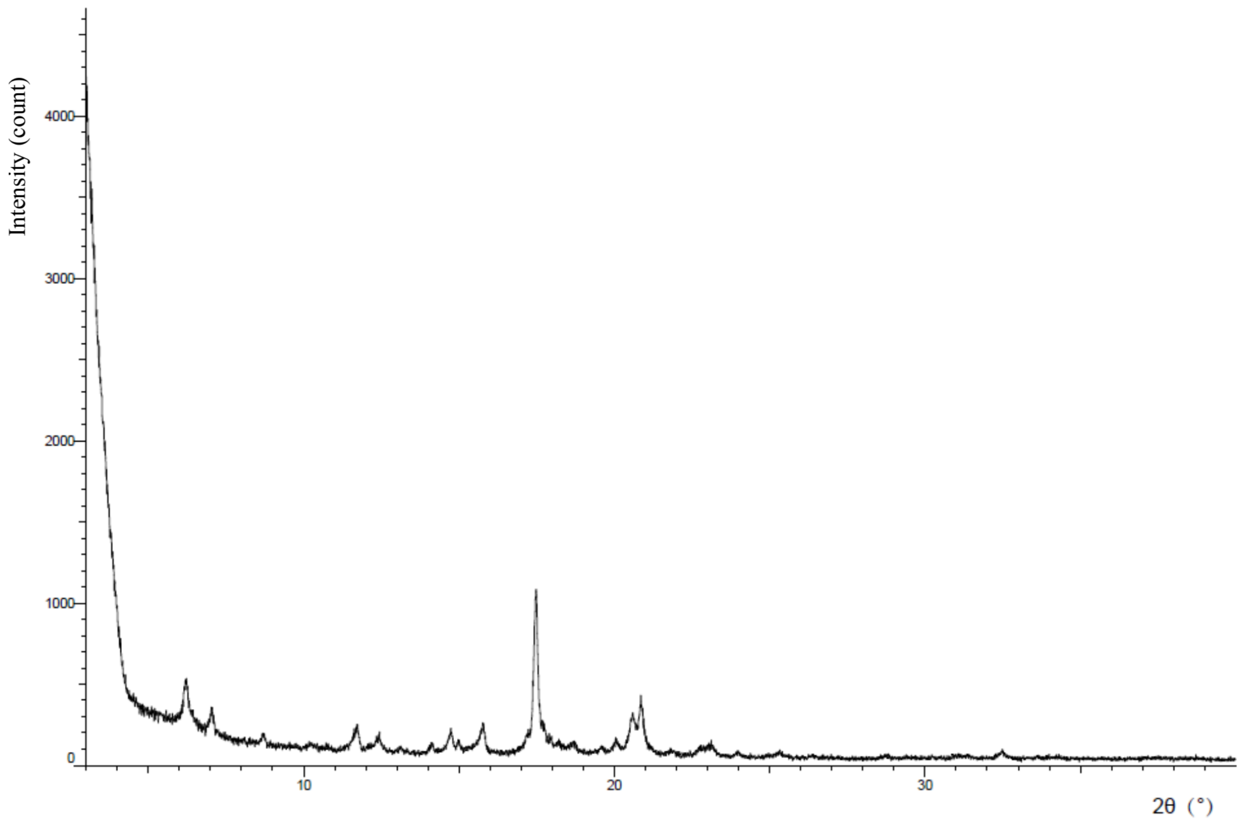
Intensity (count)
4000
3000
2000
1000
0
10
20
30
2θ (°)

FIG. 1

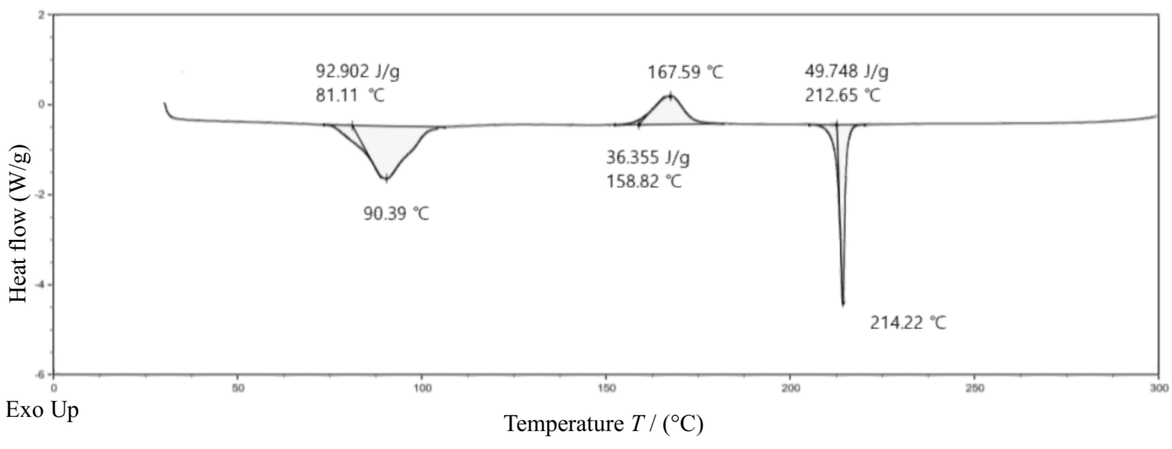
92.902 J/g
81.11 ℃
167.59 ℃
49.748 J/g
212.65 ℃
36.355 J/g
158.82 ℃
90.39 ℃
214.22 ℃
Heat flow (W/g)
2
0
-2
-4
-6
0
50
100
150
200
250
300
Exo Up
Temperature T / (°C)

FIG. 2

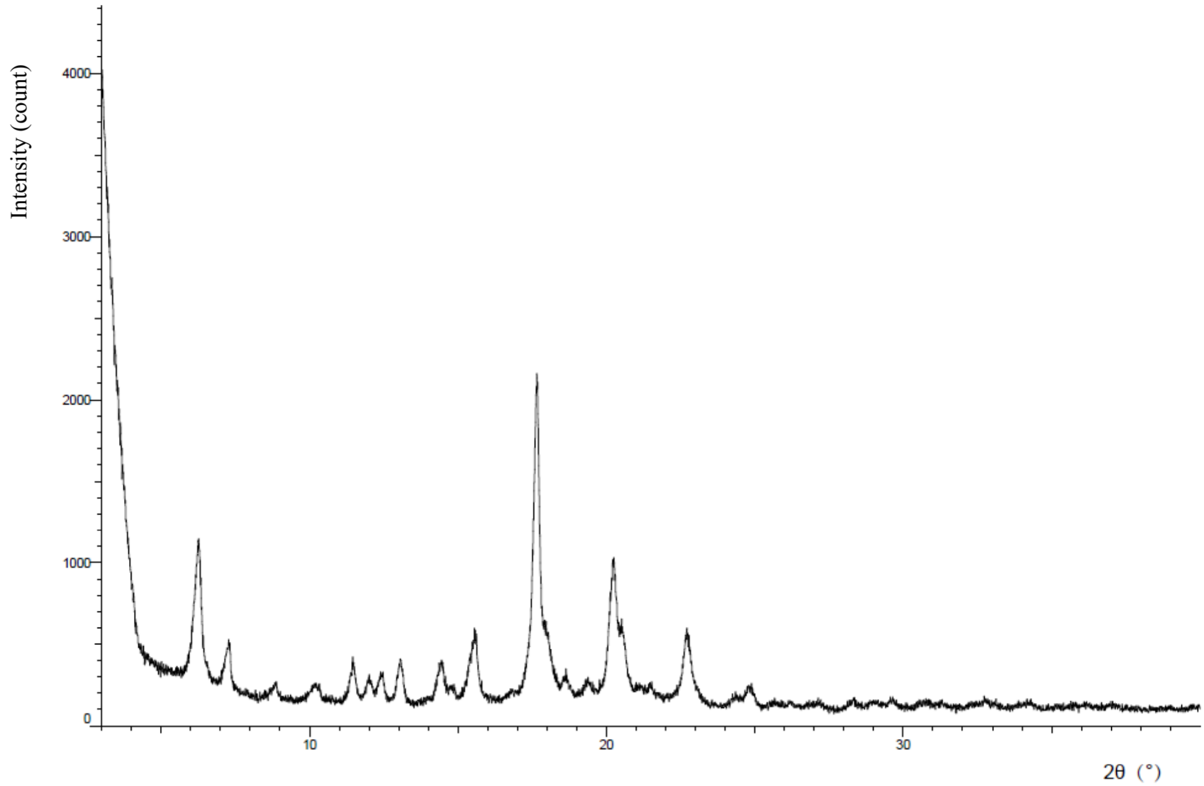
Intensity (count)
4000
3000
2000
1000
0
10
20
30
2θ (°)

FIG. 3

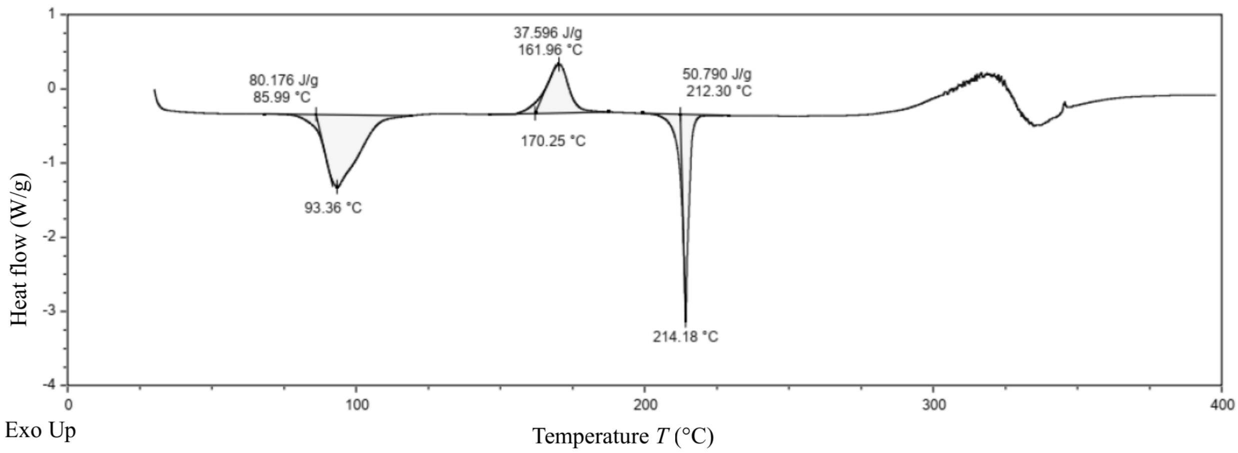
1
0
-1
-2
-3
-4
Heat flow (W/g)
80.176 J/g
85.99 °C
93.36 °C
37.596 J/g
161.96 °C
170.25 °C
50.790 J/g
212.30 °C
214.18 °C
0
100
200
300
400
Exo Up
Temperature T (°C)

FIG. 4

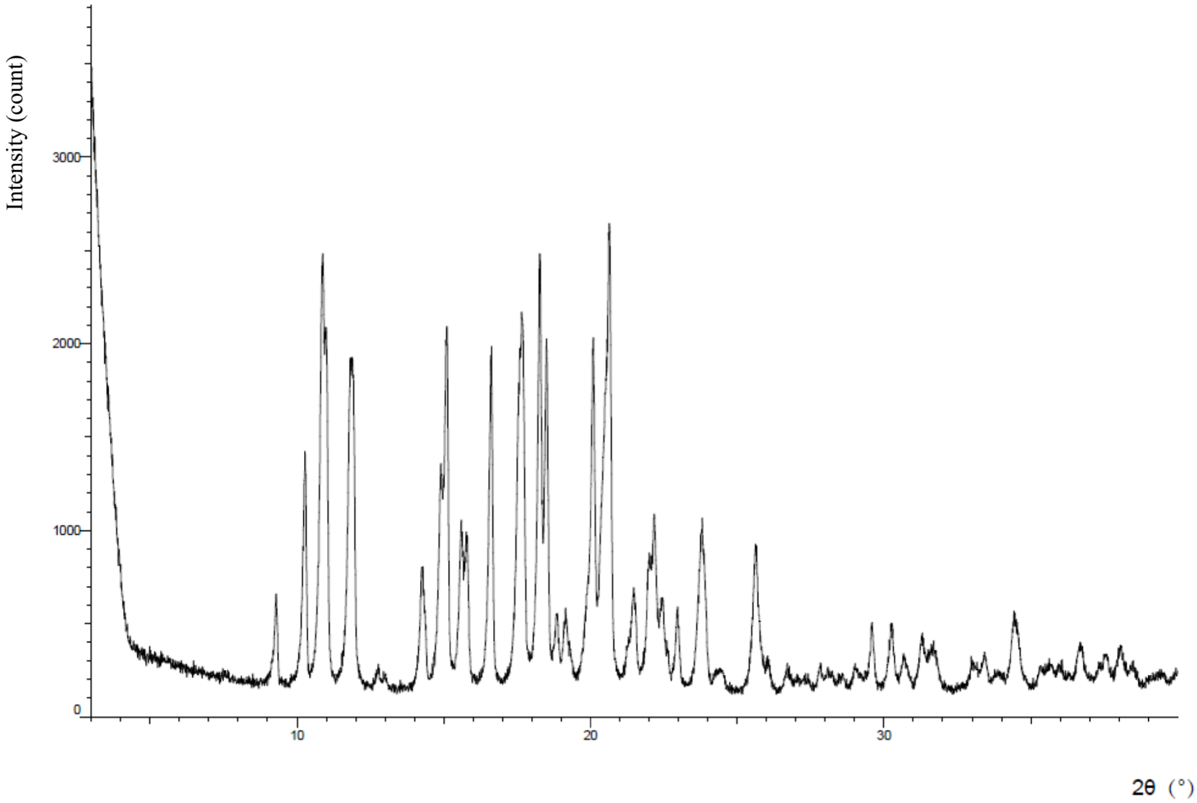
Intensity (count)
3000
2000
1000
0
10
20
30
2θ (°)

FIG. 5

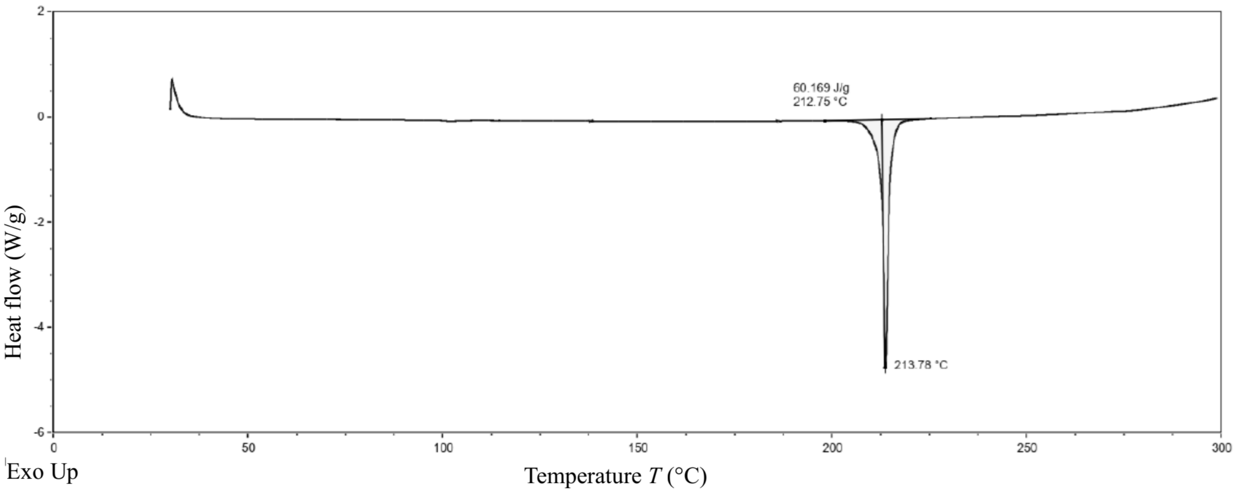
2
0
-2
-4
-6
Heat flow (W/g)
60.169 J/g
212.75 °C
213.78 °C
0
50
100
150
200
250
300
Exo Up
Temperature T (°C)

FIG. 6

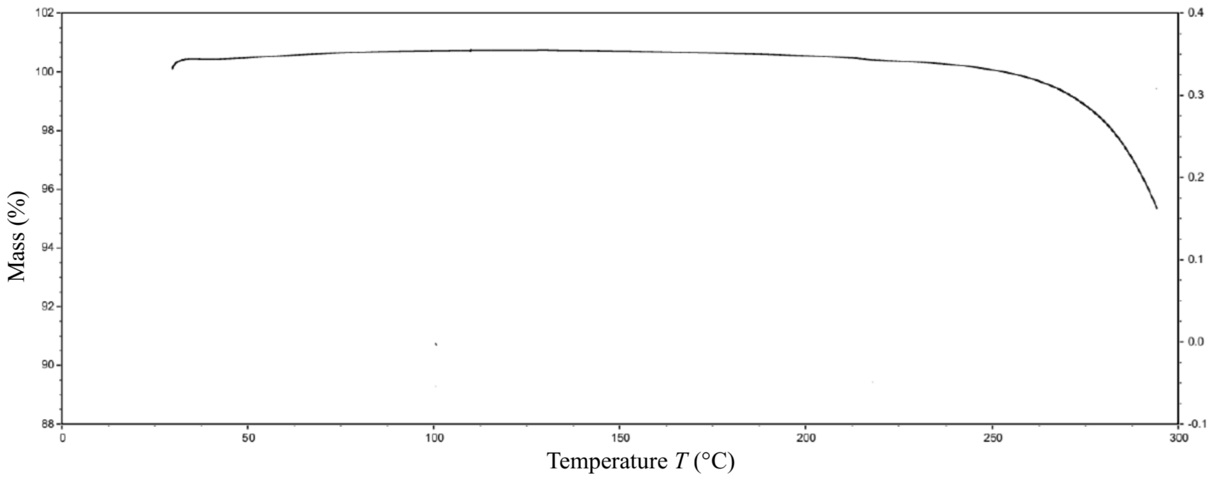
Mass (%)
Temperature T (°C)
102
100
98
96
94
92
90
88
0
50
100
150
200
250
300
0.4
0.3
0.2
0.1
0.0
-0.1

FIG. 7

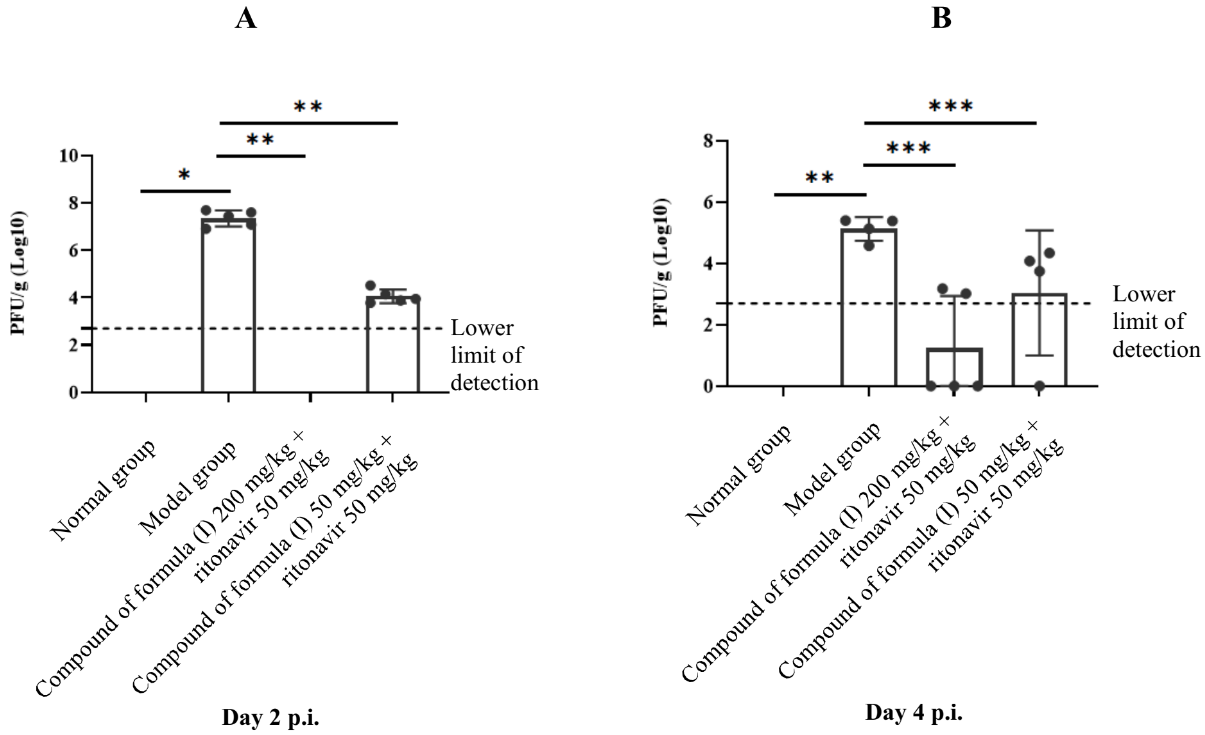
A
B
PFU/g (Log10)
Lower limit of detection
Normal group
Model group
Compound of formula (I) 200 mg/kg + ritonavir 50 mg/kg
Compound of formula (I) 50 mg/kg + ritonavir 50 mg/kg
Day 2 p.i.
Day 4 p.i.

FIG. 8

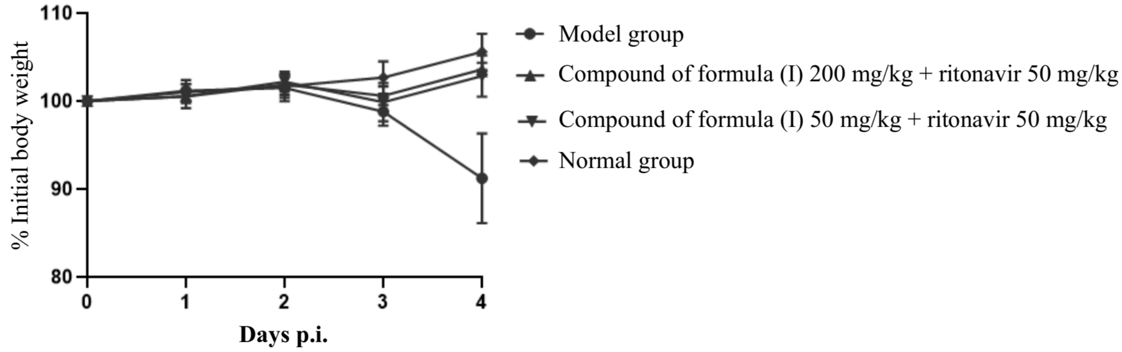

110
100
90
80
% Initial body weight
0
1
2
3
4
Days p.i.
Model group
Compound of formula (I) 200 mg/kg + ritonavir 50 mg/kg
Compound of formula (I) 50 mg/kg + ritonavir 50 mg/kg
Normal group


FIG. 9

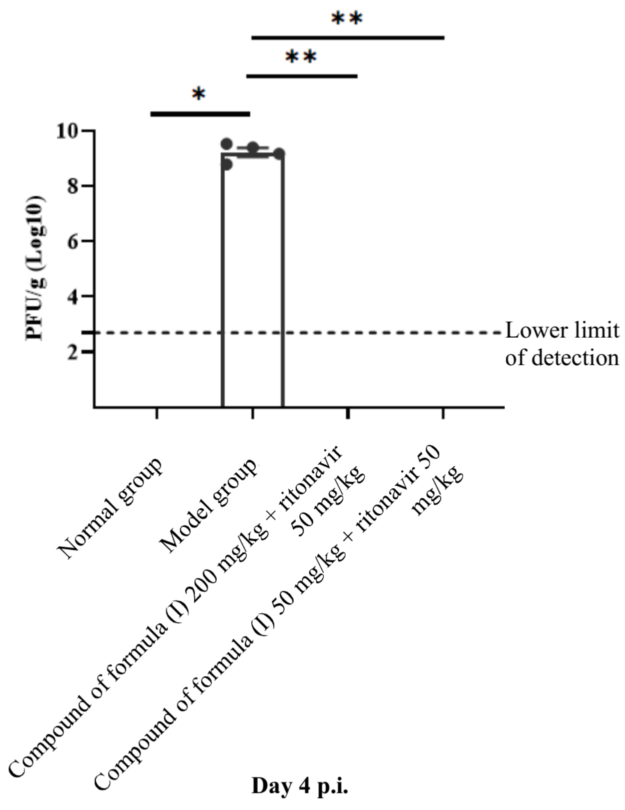

*
**
**
10
8
6
4
2
PFU/g (Log10)
Lower limit of detection
Normal group
Model group
Compound of formula (I) 200 mg/kg + ritonavir 50 mg/kg
Compound of formula (I) 50 mg/kg + ritonavir 50 mg/kg
Day 4 p.i.


FIG. 10

## INTERNATIONAL SEARCH REPORT

International application No. **PCT/CN2024/072253**

### A. CLASSIFICATION OF SUBJECT MATTER

C07K 5/027(2006.01)i; C07D285/01(2006.01)i; C07D417/14(2006.01)i; C07C51/43(2006.01)i; A61K31/381(2006.01)i; A61K31/41(2006.01)i; A61P31/14(2006.01)i

According to International Patent Classification (IPC) or to both national classification and IPC

### B. FIELDS SEARCHED

Minimum documentation searched (classification system followed by classification symbols)

IPC: C07K; C07D; C07C; A61K; A61P

Documentation searched other than minimum documentation to the extent that such documents are included in the fields searched

Electronic data base consulted during the international search (name of data base and, where practicable, search terms used)

CNABS, CNTXT, DWPI, VEN, SIPOABS, WPABSC, ENTXTC, CNKI, 万方数据资源系统, WANFANG DATA RESOURCE SYSTEM, PubMed, ISI web of knowledge, STN, Elsevier Science: 海南先声药业, 张磊, 周峰, 宋薇, 李因强, 崔楠, 高晓芳, 蒋蕾, 唐任宏, 结晶, 3CL蛋白酶, 抑制剂, 冠状病毒, 肠病毒, 螺环化合物, 式(I)化合物, 晶型, 衍射, 甲基叔丁基醚, 2-甲基四氢呋喃, DSC, 20, XRPD, 药物组合, SMA, SMB, crystalline, 3CL protease, inhibitor, coronavirus, enterovirus, spirocyclic compound, compound of formula(I), crystalline form, diffraction, methyl tert-butyl ether, 2-methyltetrahydrofuran, pharmaceutical combination

### C. DOCUMENTS CONSIDERED TO BE RELEVANT

| Category* | Citation of document, with indication, where appropriate, of the relevant passages | Relevant to claim No. |
|---|---|---|
| A | CN 113444144 A (ZHOU LONGXING et al.) 28 September 2021 (2021-09-28)<br>abstract and claims 1-5 | 1-15 |
| A | CN 114426568 A (ANDIKANG (WUXI) BIOLOGICAL TECHNOLOGY CO., LTD.) 03 May 2022 (2022-05-03)<br>abstract and claims 1-9 | 1-15 |
| A | US 2021355111 A1 (PARDES BIOSCIENCES, INC.) 18 November 2021 (2021-11-18)<br>abstract and claims 1-9 | 1-15 |
| A | Chamandi S. Dampalla, et al. "Structure-Guided Design of Potent Spirocyclic Inhibitors of Severe Acute Respiratory Syndrome Coronavirus-2 3C-Like Protease"<br>*Journal of Medicinal Chemistry,* Vol. 65, No. 11, 09 June 2022 (2022-06-09), 7818-7832<br>abstract and figures 1-2 | 1-15 |

☑ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 February 2024** | **24 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/072253

**C. DOCUMENTS CONSIDERED TO BE RELEVANT**

| Category* | Citation of document, with indication, where appropriate, of the relevant passages | Relevant to claim No. |
|---|---|---|
| A | Sherif S Ragab, et al. "Design, Synthesis, Antiviral Evaluation, and Molecular Dynamics Simulation Studies of New Spirocyclic Thiopyrimidinones as Anti HCoV-229E" *Chemistry Biodiversity,* Vol. 19, No. 10, 31 October 2022 (2022-10-31), 1-13<br>abstract, and solutions 3 and 5 | 1-15 |

Form PCT/ISA/210 (second sheet) (July 2022)

**INTERNATIONAL SEARCH REPORT**
**Information on patent family members**

International application No.
**PCT/CN2024/072253**

| Patent document cited in search report | Publication date (day/month/year) | Patent family member(s) | Publication date (day/month/year) |
|---|---|---|---|
| CN 113444144 A | 28 September 2021 | None | |
| CN 114426568 A | 03 May 2022 | None | |
| US 2021355111 A1 | 18 November 2021 | US 2022324844 A1 | 13 October 2022 |
| | | US 11124497 B1 | 21 September 2021 |
| | | US 11312704 B2 | 26 April 2022 |
| | | US 2022402896 A1 | 22 December 2022 |
| | | US 2022162194 A1 | 26 May 2022 |
| | | US 11472793 B2 | 18 October 2022 |
| | | US 2023192663 A1 | 22 June 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310064780